# EUROPEAN PATENT SPECIFICATION

(11) **EP 2 566 399 B1**
(45) Date of publication and mention of the grant of the patent: **21.11.2018**
(21) Application number: 11778387.8
(22) Date of filing: 05.05.2011
(51) Int. Cl.: A61B 17/06, A61B 17/03, D02J 3/00, A61B 17/064

(54) **SURFACE TEXTURE CONFIGURATION FOR SELF-RETAINING SUTURES**
OBERFLÄCHENTEXTURKONFIGURATION FÜR SELBSTHALTENDE NÄHTE
CONFIGURATION DE TEXTURE SUPERFICIELLE POUR SUTURES À AUTO-RETENUE

(30) Priority: 05.05.2010 US 331629 P
(43) Date of publication of application: 13.03.2013
(73) Proprietor: Ethicon LLC, San Lorenzo, PR 00754 (US)
(72) Inventor: HUNTER, William, L., Vancouver British Columbia V6A 1B6 (CA); GROSS, Jeffrey, M., Encinitas, CA 92024 (US); AVELAR, Rui, Vancouver British Columbia V6J 2W7 (CA)
(74) Representative: Small, Gary James
(86) International application number: PCT/US2011/035431
(87) International publication number: WO 2011/140400

(56) References cited:
- WO-A2-2004/030705
- WO-A2-2009/129251
- US-A- 5 931 855
- US-A1- 2007 027 475
- US-A1- 2007 208 377
- US-A1- 2009 012 560
- US-A1- 2009 143 819

## Description

### FIELD OF INVENTION

The present invention relates generally to self-retaining systems for surgical procedures.

### BACKGROUND OF INVENTION

Wound closure devices such as sutures, staples and tacks have been widely used in superficial and deep surgical procedures in humans and animals for closing wounds, repairing traumatic injuries or defects, joining tissues together (bringing severed tissues into approximation, closing an anatomical space, affixing single or multiple tissue layers together, creating an anastomosis between two hollow/luminal structures, adjoining tissues, attaching or reattaching tissues to their proper anatomical location), attaching foreign elements to tissues (affixing medical implants, devices, prostheses and other functional or supportive devices), and for repositioning tissues to new anatomical locations (repairs, tissue elevations, tissue grafting and related procedures) to name but a few examples.

Sutures are often used as wound closure devices. Sutures typically consist of a filamentous suture thread attached to a needle with a sharp point. Suture threads can be made from a wide variety of materials including bioabsorbable (i.e., that break down completely in the body over time), or non-absorbable (permanent; non-degradable) materials. Absorbable sutures have been found to be particularly useful in situations where suture removal might jeopardize the repair or where the natural healing process renders the support provided by the suture material unnecessary after wound healing has been completed; as in, for example, completing an uncomplicated skin closure. Non-degradable (non-absorbable) sutures are used in wounds where healing may be expected to be protracted or where the suture material is needed to provide physical support to the wound for long periods of time; as in, for example, deep tissue repairs, high tension wounds, many orthopedic repairs and some types of surgical anastomosis. Also, a wide variety of surgical needles are available, and the shape and size of the needle body and the configuration of the needle tip is typically selected based upon the needs of the particular application.

To use an ordinary suture, the suture needle is advanced through the desired tissue on one side of the wound and then through the adjacent side of the wound. The suture is then formed into a "loop" which is completed by tying a knot in the suture to hold the wound closed. Knot tying takes time and causes a range of complications, including, but not limited to (i) spitting (a condition where the suture, usually a knot) pushes through the skin after a subcutaneous closure), (ii) infection (bacteria are often able to attach and grow in the spaces created by a knot), (iii) bulk/mass (a significant amount of suture material left in a wound is the portion that constitutes the knot), (iv) slippage (knots can slip or come untied), and (v) irritation (knots serve as a bulk "foreign body" in a wound). Suture loops associated with knot tying may lead to ischemia (knots can create tension points that can strangulate tissue and limit blood flow to the region) and increased risk of dehiscence or rupture at the surgical wound. Knot tying is also labor intensive and can constitute a significant percentage of the time spent closing a surgical wound. Additional operative procedure time is not only bad for the patient (complication rates rise with time spent under anesthesia), but it also adds to the overall cost of the operation (many surgical procedures are estimated to cost between $15 and $30 per minute of operating time).

Self-retaining sutures (including one-way suture and barbed sutures) differ from conventional sutures in that self-retaining sutures possess numerous tissue retainers (such as barbs) which anchor the self-retaining suture into the tissue following deployment and resist movement of the suture in a direction opposite to that in which the retainers face, thereby eliminating the need to tie knots to affix adjacent tissues together (a "knotless" closure). Knotless tissue-approximating devices having barbs have been previously described in, for example, U.S. Pat. No. 5,374,268, disclosing armed anchors having barb-like projections, while suture assemblies having barbed lateral members have been described in U.S. Pat. Nos. 5,584,859 and 6,264,675. Sutures having a plurality of barbs positioned along a greater portion of the suture are described in U.S. Pat No. 5,931,855, which discloses a unidirectional barbed suture, and U.S. Pat. No. 6,241,747, which discloses a bidirectional barbed suture. Methods and apparatus for forming barbs on sutures have been described in, for example, U.S. Pat. Nos. 6,848,152. Self-retaining systems for wound closure also result in better approximation of the wound edges, evenly distribute the tension along the length of the wound (reducing areas of tension that can break or lead to ischemia), decrease the bulk of suture material remaining in the wound (by eliminating knots) and reduce spitting (the extrusion of suture material - typically knots - through the surface of the skin. All of these features are thought to reduce scarring, improve cosmesis, and increase wound strength relative to wound closures using plain sutures or staples. Thus, self-retaining sutures, because such sutures avoid knot tying, allow patients to experience an improved clinical outcome, and also save time and costs associated with extended surgeries and follow-up treatments.

US patent application publication US 2009/0143819 A1 discloses self-retaining sutures comprising tissue retainers adapted to resist movement of the suture in a direction other than the direction in which the suture is deployed into the tissue. Similarly, WO 2004/030705 A2 discloses a barbed suture with a plurality of barbs projecting from the body wherein each barb causes the suture to resist movement in an opposite direction from which the barb faces. WO 2009/129251 A2 discloses bi-directional retainers, each of which can be deployed in two directions, but once deployed in one direction resist motion in the opposite direction.

The ability of self-retaining sutures to anchor and hold tissues in place even in the absence of tension applied to the suture by a knot is a feature that also provides superiority over plain sutures. When closing a wound that is under tension, this advantage manifests itself in several ways: (i) self-retaining sutures have a multiplicity of retainers which can dissipate tension along the entire length of the suture (providing hundreds of "anchor" points this produces a superior cosmetic result and lessens the chance that the suture will "slip" or pull through) as opposed to knotted interrupted sutures which concentrate the tension at discrete points; (ii) complicated wound geometries can be closed (circles, arcs, jagged edges) in a uniform manner with more precision and accuracy than can be achieved with interrupted sutures; (iii) self-retaining sutures eliminate the need for a "third hand" which is often required for maintaining tension across the wound during traditional suturing and knot tying (to prevent "slippage" when tension is momentarily released during tying); (iv) self-retaining sutures are superior in procedures where knot tying is technically difficult, such as in deep wounds or laparoscopic/endoscopic procedures; and (v) self-retaining sutures can be used to approximate and hold the wound prior to definitive closure. As a result, self-retaining sutures provide easier handling in anatomically tight or deep places (such as the pelvis, abdomen and thorax) and make it easier to approximate tissues in laparoscopic/endoscopic and minimally invasive procedures; all without having to secure the closure via a knot. Greater accuracy allows self-retaining sutures to be used for more complex closures (such as those with diameter mismatches, larger defects or purse string suturing) than can be accomplished with plain sutures.

A self-retaining suture may be unidirectional, having one or more retainers oriented in one direction along the length of the suture thread; or bidirectional, typically having one or more retainers oriented in one direction along a portion of the thread, followed by one or more retainers oriented in another (often opposite) direction over a different portion of the thread (as described with barbed retainers in U.S. Pat. Nos. 5,931,855 and. 6,241,747). Although any number of sequential or intermittent configurations of retainers are possible, a common form of bidirectional self-retaining suture involves a needle at one end of a suture thread which has barbs having tips projecting "away" from the needle until the transition point (often the midpoint) of the suture is reached; at the transition point the configuration of barbs reverses itself about 180° (such that the barbs are now facing in the opposite direction) along the remaining length of the suture thread before attaching to a second needle at the opposite end (with the result that the barbs on this portion of the suture also have tips projecting "away" from the nearest needle). Projecting "away" from the needle means that the tip of the barb is further away from the needle and the portion of suture comprising the barb may be pulled more easily through tissue in the direction of the needle than in the opposite direction. Put another way, the barbs on both "halves" of a typical bidirectional self-retaining suture have tips that point towards the middle, with a transition segment (lacking barbs) interspersed between them, and with a needle attached to either end.

### SUMMARY OF INVENTION

Despite the multitude of advantages of unidirectional and bidirectional self-retaining sutures, there remains a need to improve upon the design of the suture. Specifically, several problems common to existing self-retaining sutures can be addressed by the embodiments of this invention, including, but not limited to: (i) retainers or barbs that are fragile and break or too flexible and bend back, or do not stand proud due to an insufficient ability of the material to plastically deform and as such do not properly engage when deployed in tissue; (ii) inadequate "hold" provided by the retainers for some surgical procedures; resulting in retainers or barbs do not sufficiently anchor in the surrounding tissue and "pull through;" (iii) insufficient contact between the retainers and the surrounding tissue (often occurring when the thread diameter is too small relative to the diameter of the hole created by a larger needle; this limits the ability of the retainers to contact and "grip" the surrounding tissue); (iv) breakage of the self-retaining suture during tensioning and wound approximation; (v) rotation and slippage of the retainers after deployment; and (vi) the difficulty of creating barbs on sutures of small diameter such as 6-0, 8-0, 10-0 and below. Furthermore, the creation and or deployment of retainer features of self- retaining sutures may be difficult to achieve without impairing the tensile strength of the suture.

Thus, it would be desirable to provide improved self-retaining sutures which have enhanced ability to anchor into the surrounding tissue, enhanced tissue holding capabilities, enhanced maximum load, and enhanced clinical performance. It would further be desirable to provide improved methods for making self-retaining sutures.

Accordingly, the present invention provides, improved self-retaining sutures which have enhanced ability to anchor into the surrounding tissue, enhanced tissue holding capabilities, enhanced maximum load, and enhanced clinical performance. Also disclosed are methods for making such self-retaining sutures.

The present invention provides suture having surface microtexture and/or nanotexture. Also disclosed are methods for making such suture.

The present invention provides self-retaining suture having surface microtexture and/or nanotexture which has an asymmetric effect on the resistance of the suture to passing through tissue in one direction compared to the other direction.

In accordance with another aspect, the present invention provides self-retaining suture having directional surface microtexture and/or nanotexture effective to secure the suture to tissue in at least one direction in the absence of macro tissue retainers such as barbs.

In accordance with another aspect, the present invention provides self- retaining suture having directional surface microtexture and/or nanotexture effective to augment the securing of the suture to tissue in at least one direction in conjunction with tissue retainers such as barbs.

The present invention provides self-retaining suture having surface microtexture and/or nanotexture which has an asymmetric effect on the resistance of the suture to passing through tissue in one direction compared to the other direction without adversely affecting the tensile strength of the suture.

In accordance with another aspect, the present invention provides self-retaining suture having surface microtexture and/or nanotexture which has an asymmetric effect on the resistance of the suture to passing through tissue in one direction compared to the other direction and which can be readily formed on small diameter sutures of sizes smaller than 4-0, 6-0, 7-0, 8-0, 9-0, 10-0 and 11-0.

According to the invention, there is provided a tissue retaining device having a flexible elongated suture thread having a surface, a longitudinal axis, a deployment direction along the longitudinal axis and a reverse direction, opposite to the deployment direction along the longitudinal axis; the suture thread having distributed on at least a portion of the surface thereof a plurality of textural features, each of the textural features comprising a top surface approximately parallel to the surface of the suture having a height of between 500nm and 10µm; and wherein the plurality of textural features cause the flexible elongated suture thread to have a greater resistance to movement through tissue in the reverse direction than in the deployment direction. The plurality of textural features includes one or more chevrons. The textural features may be asymmetrical with respect to the longitudinal axis of the suture, thread thereby causing the flexible elongated suture thread to have a greater resistance to movement through tissue in the reverse direction than in the deployment direction. The textural features include one or more chevrons and further may include one or more textural features selected from the group consisting of: ridges, grooves, columns, and pits.

In some of the foregoing embodiments, the suture thread has a first end, a second end, a periphery, and there are a plurality of retainers projecting from the periphery of the body, the plurality of retainers extending along a portion of the suture thread and oriented in one direction; and the textural features are arranged in the same portion of the suture thread.

In some others of the foregoing embodiments, there are a first plurality of retainers extending along a first portion of the suture thread and oriented in one direction and a second plurality of retainers extending along a second portion of the suture thread and oriented in an opposite direction. In these embodiments, the textural features may be arranged in a first orientation in the first portion of the suture thread and a second orientation, different than the first orientation, in the second portion of the suture thread.

In yet others of the foregoing embodiments, the suture thread has a first end, a second end, a periphery, and a plurality of retainers projecting from the periphery of the body, each retainer having a tissue-retaining surface oriented at an acute angle to the suture thread, a first plurality of the retainers extending along a first portion of the suture thread and being oriented in one direction and a second plurality of the retainers extending along a second portion of the suture thread and being oriented in an opposite direction. The textural features in these embodiments may be arranged on the tissue-retaining surface of the retainers and are adapted to augment engagement of tissue by the tissue-retaining surfaces.

In yet other of the foregoing embodiments, the suture thread has a first end, a second end, a periphery, and a plurality of reconfigurable devices projecting from the periphery of the body, each of the plurality of reconfigurable devices having a first configuration when the suture thread is deployed in a deployment direction in which a first surface is outermost and a second configuration when the suture thread is deployed in a reverse direction in which a second surface is outermost. In these embodiments, the textural features may be arranged on the second surface of each of the plurality of reconfigurable devices and are adapted to augment engagement of tissue by said second surface.

In some other embodiments of the invention, there is provided a tissue retaining device having a flexible elongated suture thread having a body, first and second ends, a surface, and a longitudinal axis, the suture thread having distributed on at least a portion of the surface thereof a plurality of textural features, each of the textural features comprising a top surface (340) approximately parallel to the surface of the suture having a height of between 500nm and 10µm; and wherein the plurality of textural features causes the flexible elongated suture thread to resist movement through tissue more in the reverse direction than in the deployment direction. The textural features include one or more chevrons. The plurality of textural features may be selected from the group consisting of ridges, grooves, columns, chevrons, and pits, they may be oriented parallel to the longitudinal axis of the suture thread, and/or they may be distributed on a portion of the suture thread surface. The device may further include at least one retainer on the portion of the suture thread surface, the at least one retainer being a cut at an acute angle into the suture thread body and including a variable-thickness tissue-penetrating edge oriented away from the first end of the suture thread. In some of these embodiments, the tissue retaining device may further include a plurality of retainers on the elongated body, each retainer having a tissue-penetrating edge oriented away from the first end and yielding toward the suture body during movement of the suture through tissue in a direction of deployment of the first end, and resisting movement of the suture, when in tissue, in a direction substantially opposite the direction of deployment of the first end. In yet others of these embodiments, the tissue retaining device may further include a plurality of first retainers disposed on the elongated body proximal to the first end, each first retainer having a tissue-penetrating edge oriented away from the first end and yielding toward the suture body during movement of the suture through tissue in a direction of deployment of the first end, and resisting movement of the suture, when in tissue, in a direction substantially opposite the direction of deployment of the first end; and a plurality of second retainers disposed on the elongated body proximal to the second end, each second retainer having a tissue- penetrating edge oriented away from the second end and yielding toward the suture body during movement of the suture through tissue in a direction of deployment of the second end, and resisting movement of the suture, when in tissue, in a direction substantially opposite the direction of deployment of the second end. The pluralities of first and second retainers may be separated by a retainer-free portion of the suture thread.

The details of one or more embodiments are set forth in the description below. Other features, objects and advantages will be apparent from the description, the drawings, and the claims.

### BRIEF DESCRIPTION OF THE DRAWINGS

Features of the invention, its nature and various advantages will be apparent from the accompanying drawings and the following detailed description of various embodiments of the disclosure and various embodiments according to the invention.
FIG. 1A shows a self-retaining suture system comprising a suture thread having directional surface microtexture and/or nanotexture.
FIGS. 1B, 1C and 1D show enlarged views of the surface of the suture thread of FIG. 1A in different portions.
FIG. 1E is a perspective view of a unidirectional self-retaining suture.
FIGS. 1F-1H are views of alternative tissue anchors for the unidirectional self-retaining suture of FIG. 1E
FIG. 1I shows a suture filament associated with a pledget.
FIG. 2A shows a segment of a suture filament having directional surface microtexture and/or nanotexture.
FIG. 2B shows an example of a feature of a directional surface microtexture and/or nanotexture.
FIG. 3A shows an enlarged view of a portion of the surface of the suture filament of FIG. 2 in accordance with an embodiment of the present invention.
FIG. 3B shows an enlarged sectional view of a portion of the surface of the suture filament shown in FIG. 3A.
FIG. 4A shows an enlarged view of a portion of the surface of the suture filament of FIG. 2.
FIG. 4B shows an enlarged sectional view of the portion of the surface of the suture filament shown in FIG. 4A.
FIG. 5A shows an enlarged view of a portion of the surface of the suture filament of FIG. 2.
FIG. 5B shows an enlarged sectional view of the portion of the surface of the suture filament shown in FIG. 5A.
FIG. 6A shows an enlarged view of a portion of the surface of the suture filament of FIG. 2.
FIG. 6B shows an enlarged sectional view of the portion of the surface of the suture filament shown in FIG. 6A.
FIG. 7A shows an enlarged view of a portion of the surface of the suture filament of FIG. 2.
FIG. 7B shows an enlarged sectional view of the portion of the surface of the suture filament shown in FIG. 7A.
FIG. 8A shows an enlarged view of a portion of the surface of the suture filament of FIG. 2.
FIG. 8B shows an enlarged sectional view of the portion of the surface of the suture filament shown in FIG. 7A.
FIG. 9A shows an enlarged view of a portion of an alternative microtexture/nanotexture.
FIG. 9B shows an enlarged sectional view of the portion of the microtexture/nanotexture shown in FIG. 9A.
FIGS. 9C-9E show views of a suture thread including reconfigurable surfaces provided in part with the microtexture/nanotexture shown in FIGS. 9A and 9B.
FIG. 9F shows a self-retaining suture thread provided in part with the microtexture/nanotexture shown in FIGS. 9A and 9B.
FIG. 10A shows a view of a portion of a self-retaining suture having microtexture/nanotexture.
FIGS. 10B and 10C show alternate views of the suture shown in FIG. 10A.

### DETAILED DESCRIPTION

### Definitions

Definitions of certain terms that may be used hereinafter include the following.

"Self-retaining system" refers to a self-retaining suture together with devices for deploying the suture into tissue. Such deployment devices include, without limitation, suture needles and other deployment devices as well as sufficiently rigid and sharp ends on the suture itself to penetrate tissue.

"Self-retaining suture" refers to a suture that includes features on the suture filament for engaging tissue without the need for a knot or suture anchor. As used herein the features include, for example, textural features.

"Tissue retainer" (or simply "retainer") refers to a physical feature of a suture filament which is adapted to mechanically engage tissue and resist movement of the suture in at least one axial directions. By way of example only, tissue retainer or retainers can include one or more of, hooks, projections, barbs, darts, extensions, bulges, anchors, protuberances, spurs, bumps, points, cogs, tissue engagers, traction devices, and the like. In certain configurations, tissue retainers are adapted to engage tissue to resist movement of the suture in a direction other than the direction in which the suture is deployed into the tissue by the surgeon, by being oriented to substantially face the deployment direction. In some embodiments the retainers lie flat when pulled in the deployment direction and open or "fan out" when pulled in a direction contrary to the deployment direction. As the tissue-penetrating end of each retainer faces away from the deployment direction when moving through tissue during deployment, the tissue retainers should not catch or grab tissue during this phase. Once the self-retaining suture has been deployed, a force exerted in another direction (often substantially opposite to the deployment direction) causes the retainers to be displaced from the deployment position (i.e. resting substantially along the suture body), forces the retainer ends to open (or "fan out") from the suture body in a manner that catches and penetrates into the surrounding tissue, and results in tissue being caught between the tissue-retaining surfaces of retainer and the suture body, which surfaces together form an acute angle; thereby "anchoring" or affixing the self-retaining suture in place. Each retainer may hold tissue and prevent movement of the suture in a direction against the deployment direction unless and until the retainer loses integrity (by, for example, bending back or breaking). In certain other configurations, the tissue retainer may be configured or combined with other tissue retainers to resist motion of the suture filament in both directions. Typically a suture having such retainers is deployed through a device such as a cannula or needle which prevents contact between the retainers and the tissue until the suture is in the desired location.

"Retainer configurations" refers to configurations of tissue retainers and can include features such as size, shape, flexibility, surface characteristics, and so forth. These are sometimes also referred to as "barb configurations".

"Bidirectional suture" refers to a self-retaining suture having retainers oriented in one direction at one end and retainers oriented in the other direction at the other end. A bidirectional suture is typically armed with a needle at each end of the suture thread. Many bidirectional sutures have a transition segment located between the two barb orientations.

"Transition segment" refers to a retainer-free (barb-free) portion of a bidirectional suture located between a first set of retainers oriented in one direction and a second set of retainers oriented in another direction. The transition segment can be at about the midpoint of the self-retaining suture, or closer to one end of the self-retaining suture to form an asymmetrical self-retaining suture system.

"Suture thread" refers to the filamentary body component of the suture. The suture thread may be a monofilament, or made of multiple filaments as in a braided suture. The suture thread may be made of any suitable biocompatible material, and may be further treated with any suitable biocompatible material, whether to enhance the sutures' strength, resilience, longevity, or other qualities, or to equip the sutures to fulfill additional functions besides joining tissues together, repositioning tissues, or attaching foreign elements to tissues.

"Monofilament suture" refers to a suture comprising a monofilamentary suture thread.

"Braided suture" refers to a suture comprising a multifilamentary suture thread. The filaments in such suture threads are typically braided, twisted, or woven together.

"Degradable (also referred to as "biodegradable" or "bioabsorbable") suture" refers to a suture which, after introduction into a tissue is broken down and absorbed by the body. Typically, the degradation process is at least partially performed in a biological system. "Degradation" refers to a chain scission process by which a polymer chain is cleaved into oligomers and monomers. Chain scission may occur through various mechanisms, including, for example, by chemical reaction (e.g., hydrolysis, oxidation/reduction, enzymatic mechanisms or a combination or these) or by a thermal or photolytic process. Polymer degradation may be characterized, for example, using gel permeation chromatography (GPC), which monitors the polymer molecular mass changes during erosion and breakdown. Degradable suture material may include polymers such as catgut, polyglycolic acid, lactic acid polymers, polyether-esters (e.g., copolymers of polyglycolide with polyglycols, polyglycolide with polyethers, polylactic acid with polyglycols or polylactic acid with polyethers), copolymers of glycolide and lactide, copolymers of trimethylene carbonate and glycolide with diethylene glycol (*e*.*g*., MAXON™, Tyco Healthcare Group), terpolymer composed of glycolide, trimethylene carbonate, and dioxanone (*e*.*g*., BIOSYN™ [glycolide (60%), trimethylene carbonate (26%), and dioxanone (14%)], Tyco Healthcare Group), copolymers of glycolide, caprolactone, trimethylene carbonate, and lactide (*e*.*g*., CAPROSYN™, Tyco Healthcare Group). These sutures can be in either a braided multifilament form or a monofilament form. The polymers used in the present invention can be linear polymers, branched polymers or multi-axial polymers. Examples of multi-axial polymers used in sutures are described in U.S. Patent Application Publication Nos. 20020161168, 20040024169, and 20040116620. Degradable sutures can also include dissolvable sutures made of a dissolvable polymer, such as a polyvinyl alcohol partly deacetylated polymer, but not limited thereto. Sutures made from degradable suture material lose tensile strength as the material degrades.

"Non-degradable (also referred to as "non-absorbable") suture" refers to a suture comprising material that is not degraded by chain scission such as chemical reaction processes (e.g., hydrolysis, oxidation/reduction, enzymatic mechanisms or a combination or these) or by a thermal or photolytic process. Non-degradable suture material includes polyamide (also known as nylon, such as nylon 6 and nylon 6.6), polyethylene terephthlate, polytetrafluoroethylene, polyether-ester (such as polybutylene or polyethylene terepthalate based copolymers with polyglycols or polyethers), polyurethane, metal alloys, metal (*e*.*g*., stainless steel wire), polypropylene, polyethelene, silk, and cotton. Sutures made of non-degradable suture material are suitable for applications in which the suture is meant to remain permanently or is meant to be physically removed from the body.

Sutures materials are broadly classified as being degradable or bioabsorbable (i.e., they break down completely in the body over time), such as those composed of catgut, glycolic acid polymers and copolymers, lactic acid polymers and copolymers, and polyether-esters based copolymers such as polyglycolide or lactide copolymers with polyglycols or polyethers; or as being non-absorbable (permanent; nondegradable), such as those made of polyamide, polytetrafluoroethylene, polyethylene terephthalate, polyurethane, polyether-esters based copolymers such as polybutylene or polyethylene terephthalate with polyglycols or polyethers, metal alloys, metal (*e*.*g*., stainless steel wire), polypropylene, polyethelene, silk, and cotton. Degradable (bioabsorbable) sutures have been found to be particularly useful in situations where suture removal might jeopardize the repair or where the natural healing process renders the support provided by the suture material unnecessary after wound healing has been completed; as in, for example, completing an uncomplicated skin closure. Nondegradable (non-absorbable) sutures are used in wounds where healing may be expected to be protracted or where the suture material is needed to provide physical support to the wound for long periods of time; as in, for example, deep tissue repairs, high tension wounds, many orthopedic repairs and some types of surgical anastomoses.

Bioabsorbable sutures can be made of materials which are broken down in tissue after a given period of time, which depending on the material can be from ten days to eight weeks. The sutures are used therefore in many of the internal tissues of the body. In most cases, three weeks is sufficient for the wound to close firmly. At that time the suture is not needed any more, and the fact that it disappears is an advantage, as there is no foreign material left inside the body and no need for the patient to have the sutures removed. In rare cases, bioabsorbable sutures can cause inflammation and be rejected by the body rather than absorbed. Bioabsorbable sutures were first made from the intestines of mammals. For example, gut sutures can be made of specially prepared bovine or ovine intestine, and can be untreated (plain catgut), tanned with chromium salts to increase the suture persistence in the body (chromic catgut), or heat-treated to give more rapid absorption (fast catgut). Concern about transmitting diseases such as bovine spongiform encephalopathy, has resulted in the gut being harvested from stock which have been tested to determine that the natural polymers used as suture materials do not carry viral diseases. Bioabsorbable sutures can be made of synthetic polymer fibers, which can be monofilaments or braided.

"Suture diameter" refers to the diameter of the body of the suture. It is to be understood that a variety of suture lengths may be used with the sutures described herein and that while the term "diameter" is often associated with a circular periphery, it is to be understood herein to indicate a cross-sectional dimension associated with a periphery of any shape. Suture sizing is based upon diameter. United States Pharmacopeia ("USP") designation of suture size runs from 0 to 7 in the larger range and 1-0 to 11-0 in the smaller range; in the smaller range, the higher the value preceding the hyphenated zero, the smaller the suture diameter. The actual diameter of a suture will depend on the suture material, so that, by way of example, a suture of size 5-0 and made of collagen will have a diameter of 0.15 mm, while sutures having the same USP size designation but made of a synthetic absorbable material or a non-absorbable material will each have a diameter of 0.1 mm. The selection of suture size for a particular purpose depends upon factors such as the nature of the tissue to be sutured and the importance of cosmetic concerns; while smaller sutures may be more easily manipulated through tight surgical sites and are associated with less scarring, the tensile strength of a suture manufactured from a given material tends to decrease with decreasing size. It is to be understood that the sutures and methods of manufacturing sutures disclosed herein are suited to a variety of diameters, including without limitation USP 7, 6, 5, 4, 3, 2, 1, 0, 1-0, 2-0, 3-0, 4-0, 5-0, 6-0, 7-0, 8-0, 9-0, 10-0 and 11-0 (09, 0.8, 0.7, 0.6, 0.5, 0.4, 0.35, 0.3, 0.2, 0.15, 0.1, 0.07, 0.05, 0,04, 0.03, 0.02, 0.01 mm).

"Suture deployment end" refers to an end of the suture to be deployed into tissue; one or both ends of the suture may be suture deployment ends. The suture deployment end may be attached to a deployment device such as a suture needle, or may be sufficiently sharp and rigid to penetrate tissue on its own.

"Armed suture" refers to a suture having a suture needle on at least one suture deployment end.

"Needle attachment" refers to the attachment of a needle to a suture requiring same for deployment into tissue, and can include methods such as crimping, swaging, using adhesives, and so forth. The suture thread is attached to the suture needle using methods such as crimping, swaging and adhesives. Attachment of sutures and surgical needles is described in U.S. Patent Nos. 3,981,307, 5,084,063, 5,102,418, 5,123,911, 5,500,991, 5,722,991, 6,012,216, and 6,163,948, and U.S. Patent Application Publication No. US 2004/0088003). The point of attachment of the suture to the needle is known as the swage.

"Suture needle" refers to needles used to deploy sutures into tissue, which come in many different shapes, forms and compositions. There are two main types of needles, traumatic needles and atraumatic needles. Traumatic needles have channels or drilled ends (that is, holes or eyes) and are supplied separate from the suture thread and are threaded on site. Atraumatic needles are eyeless and are attached to the suture at the factory by swaging or other methods whereby the suture material is inserted into a channel at the blunt end of the needle which is then deformed to a final shape to hold the suture and needle together. As such, atraumatic needles do not require extra time on site for threading and the suture end at the needle attachment site is generally smaller than the needle body. In the traumatic needle, the thread comes out of the needle's hole on both sides and often the suture rips the tissues to a certain extent as it passes through. Most modern sutures are swaged atraumatic needles. Atraumatic needles may be permanently swaged to the suture or may be designed to come off the suture with a sharp straight tug. These "pop-offs" are commonly used for interrupted sutures, where each suture is only passed once and then tied. For barbed sutures that are uninterrupted, these atraumatic needles are preferred.

Suture needles may also be classified according to the geometry of the tip or point of the needle. For example, needles may be (i) "tapered" whereby the needle body is round and tapers smoothly to a point; (ii) "cutting" whereby the needle body is triangular and has a sharpened cutting edge on the inside; (iii) "reverse cutting" whereby the cutting edge is on the outside; (iv) "trocar point" or "taper cut" whereby the needle body is round and tapered, but ends in a small triangular cutting point; (v) "blunt" points for sewing friable tissues; (vi) "side cutting" or "spatula points" whereby the needle is flat on top and bottom with a cutting edge along the front to one side (these are typically used for eye surgery).

Suture needles may also be of several shapes including, (i) straight, (ii) half curved or ski, (iii) 1/4 circle, (iv) 3/8 circle, (v) 1/2 circle, (vi) 5/8 circle, (v) and compound curve.

Suturing needles are described, for example, in US Patent Nos. 6,322,581 and 6,214,030 (Mani, Inc., Japan); and 5,464,422 (W.L. Gore, Newark, DE); and 5,941,899; 5,425,746; 5,306,288 and 5,156,615 (US Surgical Corp., Norwalk, CT); and 5,312,422 (Linvatec Corp., Largo, FL); and 7,063,716 (Tyco Healthcare, North Haven, CT). Other suturing needles are described, for example, in US Patent Nos. 6,129,741; 5,897,572; 5,676,675; and 5,693,072. The sutures described herein may be deployed with a variety of needle types (including without limitation curved, straight, long, short, micro, and so forth), needle cutting surfaces (including without limitation, cutting, tapered, and so forth), and needle attachment techniques (including without limitation, drilled end, crimped, and so forth). Moreover, the sutures described herein may themselves include sufficiently rigid and sharp ends so as to dispense with the requirement for deployment needles altogether.

"Needle diameter" refers to the diameter of a suture deployment needle at the widest point of that needle. While the term "diameter" is often associated with a circular periphery, it is to be understood herein to indicate a cross-sectional dimension associated with a periphery of any shape.

"Wound closure" refers to a surgical procedure for closing of a wound. An injury, especially one in which the skin or another external or internal surface is cut, torn, pierced, or otherwise broken is known as a wound. A wound commonly occurs when the integrity of any tissue is compromised (e.g., skin breaks or burns, muscle tears, or bone fractures). A wound may be caused by an act, such as a puncture, fall, or surgical procedure; by an infectious disease; or by an underlying medical condition. Surgical wound closure facilitates the biological event of healing by joining, or closely approximating, the edges of those wounds where the tissue has been torn, cut, or otherwise separated. Surgical wound closure directly apposes or approximates the tissue layers, which serves to minimize the volume new tissue formation required to bridge the gap between the two edges of the wound. Closure can serve both functional and aesthetic purposes. These purposes include elimination of dead space by approximating the subcutaneous tissues, minimization of scar formation by careful epidermal alignment, and avoidance of a depressed scar by precise eversion of skin edges.

"Tissue elevation procedure" refers to a surgical procedure for repositioning tissue from a lower elevation to a higher elevation (i.e. moving the tissue in a direction opposite to the direction of gravity). The retaining ligaments of the face support facial soft tissue in the normal anatomic position. However, with age, gravitational effects and loss of tissue volume effect downward migration of tissue, and fat descends into the plane between the superficial and deep facial fascia, thus causing facial tissue to sag. Face-lift procedures are designed to lift these sagging tissues, and are one example of a more general class of medical procedure known as a tissue elevation procedure. More generally, a tissue elevation procedure reverses the appearance change that results from effects of aging and gravity over time, and other temporal effects that cause tissue to sag, such as genetic effects. It should be noted that tissue can also be repositioned without elevation; in some procedures tissues are repositioned laterally (away from the midline), medially (towards the midline) or inferiorly (lowered) in order to restore symmetry (i.e. repositioned such that the left and right sides of the body "match").

"Medical device" or "implant" refers to any object placed in the body for the purpose of restoring physiological function, reducing/alleviating symptoms associated with disease, and/or repairing and/or replacing damaged or diseased organs and tissues. While normally composed of biologically compatible synthetic materials (e.g., medical-grade stainless steel, titanium and other metals or polymers such as polyurethane, silicon, PLA, PLGA and other materials) that are exogenous, some medical devices and implants include materials derived from animals (e.g., "xenografts" such as whole animal organs; animal tissues such as heart valves; naturally occurring or chemically-modified molecules such as collagen, hyaluronic acid, proteins, carbohydrates and others), human donors (e.g., "allografts" such as whole organs; tissues such as bone grafts, skin grafts and others), or from the patients themselves (e.g., "autografts" such as saphenous vein grafts, skin grafts, tendon/ligament/muscle transplants). Medical devices that can be used in procedures in conjunction with the present invention include, but are not restricted to, orthopedic implants (artificial joints, ligaments and tendons; screws, plates, and other implantable hardware), dental implants, intravascular implants (arterial and venous vascular bypass grafts, hemodialysis access grafts; both autologous and synthetic), skin grafts (autologous, synthetic), tubes, drains, implantable tissue bulking agents, pumps, shunts, sealants, surgical meshes (e.g., hernia repair meshes, tissue scaffolds), fistula treatments, spinal implants (e.g., artificial intervertebral discs, spinal fusion devices, etc.) and the like.

This application also uses the terms proximal and distal in the conventional manner when describing an implant and/or suture. Thus, proximal refers to the end or side of a device or component closest to the hand operating the device, whereas distal refers to the end or side of a device furthest from the hand operating the device. For example, the needle end of a suture would conventionally be called the proximal end (it is closest to the surgeon) while the far end of the suture would be termed the distal end. For a bidirectional self-retaining suture the proximal end refers to the needle end of whichever arm is being deployed.

In the description that follows, common reference numerals are used to indicate like elements throughout the drawings and detailed description; therefore, reference numerals used in a drawing may or may not be referenced in the detailed description specific to such drawing if the associated element is described elsewhere. The first digit in a three digit reference numeral indicates the series of figures in which the referenced item first appears. Likewise the first two digits in a four digit reference numeral.

### Self-retaining Suture Having Directional Surface Microtexture

As discussed above, the present invention provides self-retaining systems for use in surgical procedures which increase the ability of the self-retaining sutures to anchor into the surrounding tissue to provide superior holding strength and improve clinical performance. In the following description, the expression "embodiment" may refer to embodiments of the disclosure which do not necessarily also form embodiments of the invention. The scope of the invention is defined in the appended claims. In accordance with one embodiment, the present invention provides a variety of surface configurations for sutures where those configurations enhance or diminish the ability of a suture to move through tissue. The surface features differ from retainers in that they provide some resistance to movement of the suture in tissue, and if sufficient force is exerted on the suture to overcome such resistance then movement may occur without necessarily causing breakage, deformation, or other destruction of surface feature integrity or, alternatively, without causing substantial tissue damage. Additionally, the resistance provided by these surface features is not necessarily against movement in a direction contrary to the deployment direction; the resistance may be against movement in other directions or it may be non-directional. Surface features include directional and non-directional microtextures and/or nanotextures. For example, in order to resist the movement of the suture through tissue, the surface may have a tire-tread appearance. The microtexture and/or nanotexture is, in some embodiments, designed to reduce the resistance to movement of the suture through tissue. The microtexture and/or nanotexture is, in some embodiments, designed to achieve a larger resistance of the suture to movement of the suture through tissue in a deployment direction as compared to a reverse direction. The microtexture and/or nanotexture can be created using a variety of methods including, for example, extrusion, laser ablation, nanomolding, chemical ablation (e.g., as done in lithography), mechanical cutting, and coining. Alternatively a material, retainer, scale, sheath, sleeve having a desired surface configuration can be secured to a suture thread.

### Self-Retaining Suture Systems

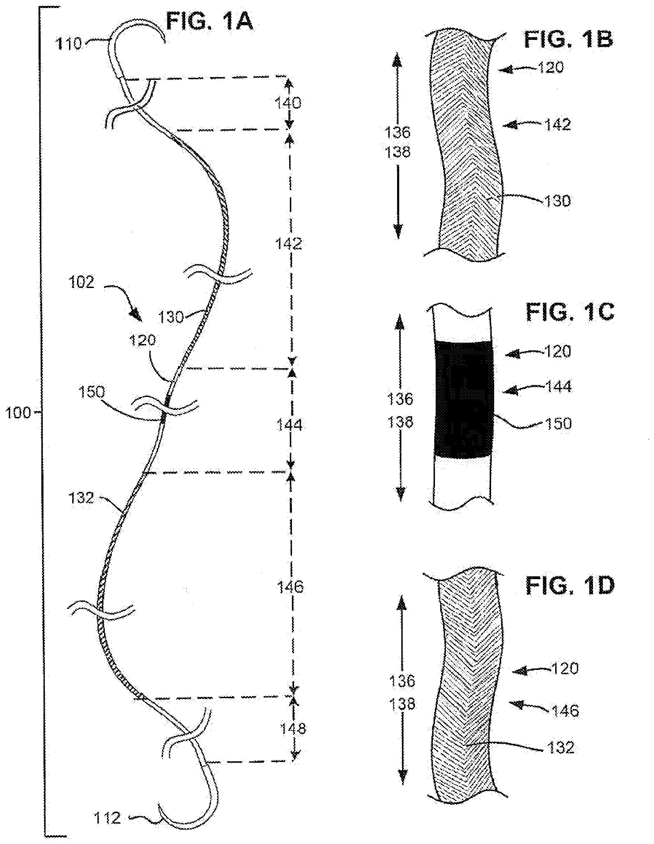

FIG. 1A illustrates a bidirectional self-retaining suture system 100. Self-retaining suture system 100 includes needles 110, 112 attached to self-retaining suture thread 102. Self-retaining suture thread 102 includes a directional microtexture and/or nanotexture 130, 132 on the surface filament 120. In lead-in region 140 of filament 120 there is no directional microtexture and/or nanotexture 130, 132. In region 142 of variable-dimension filament 120 there is a directional microtexture and/or nanotexture 130 in the form of chevron shaped grooves oriented such that the suture can be deployed in the direction of needle 110, but resists movement in the direction of needle 112. In transition region 144, there is no directional microtexture and/or nanotexture 130, 132. In region 146 of filament 120 there is a directional microtexture and/or nanotexture 132 in the form of chevron shaped grooves oriented such that the suture can be deployed in the direction of needle 112, but resists movement in the direction of needle 110. In lead-in region 148 of variable-dimension filament 120 there is no directional microtexture and/or nanotexture 130, 132.

A break is shown in each of regions 140, 142, 144, 146 and 148 to indicate that the length of each region may be varied and selected depending upon the application for which the suture is intended to be used. Although a bidirectional self-retaining suture system 100 is illustrated, the present invention includes self-retaining suture systems of a wide variety of retainer and needle configurations as described above. Likewise, although needles 110 and 112 are shown as curved needles, needles 110 and 112 can be any of the range of different surgical needles developed for use in different applications. Needles 110 and 112 may have the same configuration or different configurations.

Additionally, in many procedures it is desirable to locate the transition region in order to properly situate the transition region at the beginning of suture deployment. Thus, the filament 120 in section 144 is, in some embodiments, provided with an identifiable feature. For example, as shown in FIGS. 1A and 1C, section 144 of self-retaining suture system 100 is provided with an identifiable marker in the form of visible band 150. Band 150 represents a portion of filament 120 having a different visible characteristic than other portions of filament 120 which can thus be identified by a surgeon in order to identify and locate the transition section 144 of self-retaining suture system 100. In alternative embodiments, markers are provided on other sections of the filament and/or needles in order to identify features of the self-retaining suture system associated with the section marked. Additionally, marker differences can include different shapes, different colors, different numbers, and different letters to name a few types of markers.

FIG. 1B illustrates a magnified view of self-retaining suture thread 102 in section 142. As shown in FIG. 1B, a directional microtexture and/or nanotexture is provided on the surface of filament 120 in section 142. The directional microtexture and/or nanotexture provided in section 142 is designed and oriented such that the suture can be deployed in direction 136, but resists movement in direction 138.

FIG. 1C illustrates a magnified view of self-retaining suture thread 102 in section 144. As shown in FIG. 1C, there is no directional microtexture and/or nanotexture provided on the surface of filament 120 in section 144. Section 144 may be referred to as the transition section of self-retaining suture system 100. Section 144 may be deployed in either of the directions shown by arrows 136 and 138. In many procedures it is desirable to locate the transition region in order to properly situate the transition region at the beginning of suture deployment. Band 150 represents a portion of filament 120 having a different visible characteristic than other portions of filament 120 which can thus be identified by a surgeon in order to identify and locate the transition section 144.

FIG. ID illustrates a magnified view of self-retaining suture thread 102 in section 146. As shown in FIG. 1D, a directional microtexture and/or nanotexture is provided on the surface of filament 120 in section 146. The directional microtexture and/or nanotexture is oriented in the opposite direction from the directional microtexture and/or nanotexture provided in section 142. Thus, the directional microtexture and/or nanotexture in section 146 is designed and oriented such that the suture can be deployed in direction 138, but resists movement in direction 136. With respect to Figs. 1B and 1D, the opposite surfaces of the cylindrical suture filament 120 has similar chevron shaped grooves or other styles of microtexture and/or nanotexture. That is to say on the opposite surface of region 142 of the suture, there are chevron shaped grooves that allows the suture to be deployed in the direction of needle 110, but resists deployment of the suture in the direction of needle 112. In a preferred embodiment, the chevron shaped grooves on opposite sides do not connect with each other and are preferably separated by a channel. If these chevron shaped grooves on opposite sides of the suture connected, the area of connection could prevent motion in the direction of needle 110. Further, in another embodiment, the chevron shaped grooves can be placed on only one surface of the suture and not on an opposite surface of the suture. Similarly, chevron shaped grooves on opposite surfaces of region 146 of the suture would preferably not be connected so that motion in the direction of needle 112 would not be resisted.

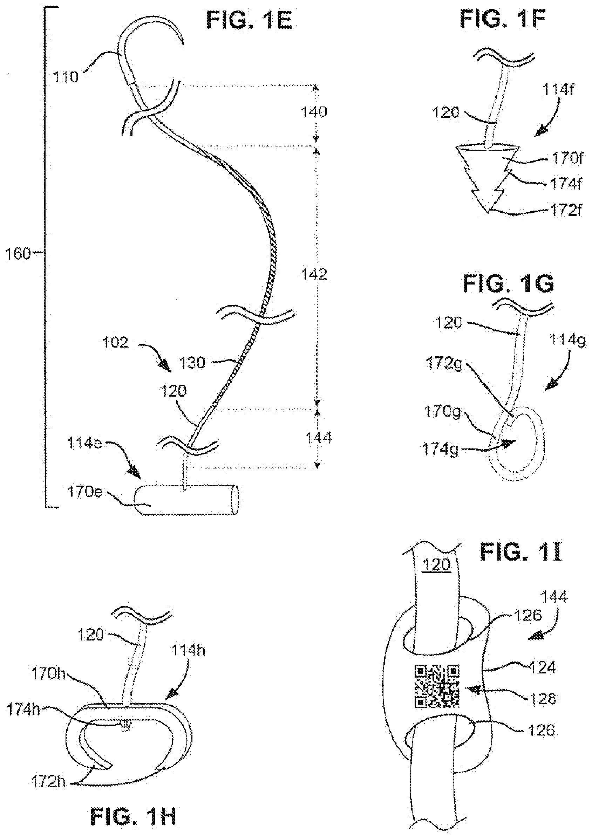

FIG. 1E illustrates an alternative embodiment of a self-retaining suture system 160. Self-retaining suture system 160 includes needle 110 and sections 140, 142 and 144 of self-retaining suture system 100 of FIG. 1A. However, self-retaining suture system 160 is a single-armed system. As shown in FIG. 1E, filament 120 terminates following section 146 in a tissue anchor 114e. Tissue anchor 114e is a device for engaging tissue and preventing filament 120 from moving through tissue in the direction of needle 110. Tissue anchor 114e is in some embodiments formed in one piece with filament 120 or formed separately and subsequently attached to filament 120. As shown in FIG. 1E, tissue anchor 114e has a bar-shaped body 170e which extends approximately perpendicular to the axis of filament 120. Bar-shaped body 170e is sufficiently long and stiff to preclude movement of filament 120 in the direction of needle 110 after tissue anchor 114e has engaged a tissue.

FIG. 1F shows an alternative anchor 114f which could be used in place of tissue anchor 114e of FIG. 1E. As shown in FIG. 1F, tissue anchor 114f comprises a conical body 170f. Conical body 170f has a pointed end 172f and tissue engaging features 174f which consist of ribs and/or barbs. Tissue anchor 114f is configured to be pushed into tissue in order to anchor filament 120 to that tissue and preclude movement of filament 120 in the direction of needle 110. Anchor 114f is, in some embodiments, formed in one piece with filament 120. In other embodiments, anchor 114f is bonded and/or mechanically fixed to suture thread 120, by, for example, welding, clipping, gluing, and/or fusing.

FIG. 1G shows an alternative anchor 114g which could be used in place of tissue anchor 114e of FIG. 1E. As shown in FIG. 1G, tissue anchor 114g comprises a loop 170g. Loop 170g is, in this embodiment, formed by folding back the end 172g of filament 120 and securing end 172g to filament 120 by welding, fusing and/or adhesive. Loop 170g is thus formed from the material of filament 120. Loop 170g has an aperture 174g through which needle 110 can pass in order to create a noose which can be used to engage tissue and preclude movement of filament 120 in the direction of needle 110.

FIG. 1H shows an alternative anchor 114h which could be used in place of tissue anchor 114e of FIG. 1E. As shown in FIG. 1H, tissue anchor 114h comprises a staple-shaped body 170h. Filament 120 passes through an aperture in anchor 114h and is secured by a crimp 174h. Staple-shaped body 170h has two points pointed end 172h which can be deformed towards each other to engage tissue and preclude movement of filament 120 in the direction of needle 110. Anchor 114h is, in some embodiments, formed in one piece with filament 120. In other embodiments, anchor 114h is formed separately from a different biocompatible material (such as steel, nitinol or titanium) and then bonded and/or mechanically fixed to suture thread 120, by, for example, welding, clipping, crimping, gluing, and/or fusing. Anchors such as anchors 170e through 170h are, in some embodiments, also provided with surface microtexture and/or nanotexture to assist in the engagement of tissue.

In alternative embodiments, a pledget can be applied to a self-retaining suture system. FIG. 1I depicts a pledget 124 located, for example in the transition zone 144 of self-retaining suture system 100 of FIG 1A. In some embodiments, pledget 124 can carry a marker/code 128 which helps identify the suture and/or properties thereof. Pledget 124 has one or more apertures 126 through which suture thread 120 can be passed as shown.. The pledget 126 can be used for locating the transition zone, for providing a stop so that the pledget can be pulled through tissue only until the pledget contacts the tissue, and/or for providing a support to tissue and organs, to name just a few uses. In alternative embodiments a pledget is formed in one piece with the suture thread or formed separately and bonded and/or mechanically fixed to suture thread 120, by, for example, welding, clipping, gluing, and/or fusing. The pledget 126 can take many forms including a wider section that can support tissue. Pledgets can similarly be used in various locations on other bidirectional or unidirectional self-retaining suture systems. Pledgets are, in some embodiments, also provided with a microtextured and or nanotextured surface.

### Suture Filaments Having Directional Surface Microtexture

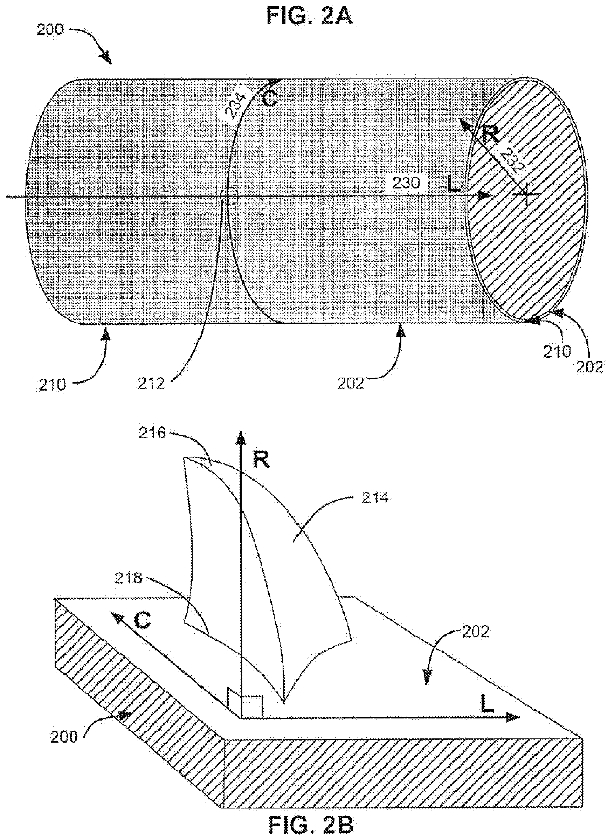

FIG. 2A shows a segment of a suture filament 200 having a filament surface 202 on which there is a directional surface microtexture and/or nanotexture 210 in accordance with an embodiment of the present invention. A representative patch 212 of the filament surface 202 on which there is a directional surface microtexture and/or nanotexture 210 is indicated by a dotted line. Arrow 230 indicates the longitudinal axis L of the suture surface 202. The longitudinal axis L is parallel to the suture surface 202 and parallel to the longitudinal axis of the suture filament 200. Arrow 234 indicates the circumferential axis C. The circumferential axis C is parallel to the suture surface 202 and perpendicular to the longitudinal axis of the suture filament 200. Arrow 232 indicates the radial axis R of the suture surface 202. The radial axis R is an axis normal to the macro filament surface 202. Suture filament 200 is, in some embodiments, incorporated into a self-retaining suture system such as shown in FIGS. 1A-1I. Furthermore, it is intended that suture filament 200 can be provided with any one or more of the microtextures and/or nanotextures described herein.

A directional surface microtexture and/or nanotexture typically includes an arrangement of textural features distributed on the surface 202 of a filament 200. The arrangement can be a regular arrangement in some embodiments and an irregular and/or random arrangement in other embodiments. The textural features are in some embodiments made by subtracting material from the surface 202 of the suture filament 200 (e.g. laser machining). In other embodiments material is added to the surface 202 of suture filament 200 to create textural features (e.g. nanomolding added material). In still other embodiments, the material at the surface 202 of suture filament 200 is treated/manipulated to create textural features (e.g. coining/imprinting). The material is in some embodiments the same as the material of the suture filament and in other embodiments is different the material of the suture filament. In other embodiments the surface 202 of the suture filament 200 is treated so that it changes shape to form textural features. In other embodiments combinations of one or more of the processes of adding material to the surface, subtracting material from the surface and treating the surface is performed in any order to create textural features.

FIG. 2B illustrates a greatly enlarged example of a feature 214 of a directional surface microtexture and/or nanotexture on a surface 202 of a suture filament. As a convention, when discussing surface microtexture and/or nanotexture on a suture filament, the width of a feature 214 of surface texture refers to the size of the feature along the radial axis C and the length of a feature of surface texture refers to the size of the feature along the longitudinal axis L. The height of a feature 214 of surface texture refers the size of the feature along the radial axis R. The top 216 of a feature 214 of surface texture is the portion of the feature 14 furthest above the surface 202 of the filament. Conversely the base 218 of a feature 214 of surface texture is the portion of the feature immediately adjacent the surface 202 of the filament.

Microtexture, as used herein, refers to a texture consisting of features (microfeatures) having one or more characteristic fixed dimensions less than about 10µm and greater than 1µm. Nanotexture conventionally refers to textures consisting of features (nanofeatures) having one or more characteristic fixed dimensions smaller than 1000nm. Such features are in some embodiments larger in one or more dimensions, for example, a groove of a microtexture can have a width and a fixed depth less than 10µm but a length much longer than 10µm. Likewise a ridge of a nanotexture can have a width and a fixed height less than 1000nm but a length much longer than 1000nm. (It is to be understood that the terms height and depth both refer to the dimension of the feature that is transversely perpendicular to the longitudinal axis of the suture, and may thus be used interchangeably herein.)

Microtextures and/or nanotextures are known to effect changes in the surface tissue interaction including for example, adhesion, wettability, tissue ingrowth, tissue engagement, chemistry, stiction and friction. A directional microtexture and/or nanotexture as used herein refers to an arrangement of microfeatures and/or nanofeatures wherein an aspect of the shape and/or orientation and/or distribution of the microfeatures and/or nanofeatures causes the surface bearing the texture to have a greater resistance to movement through tissue in one direction compared to another direction as will be described with respect to the particular embodiments of microtextures and nanotextures disclosed below. Generally, a directional microtexture and/or nanotexture will have an asymmetry with respect to an aspect of the shape and/or orientation and/or distribution of the microfeatures and/or nanofeatures. With respect to a self-retaining suture the directional microtexture and/or nanotexture causes the filament to have less resistance to passing through tissue in the forward direction along the longitudinal axis L (in the direction of deployment) compared to the reverse direction along the longitudinal axis L (against the direction of deployment).

Embodiments of the present invention include suture threads and other surgical filaments having thereon directional microtexture and/or nanotexture which causes the filament to have less resistance to passing through tissue in the forward direction along the longitudinal axis L (in the direction of deployment) compared to the reverse direction along the longitudinal axis L (against the direction of deployment). Particular embodiments of such directional microtextures and nanotextures are described below.

### Directional Surface Microtextures And Nanotextures

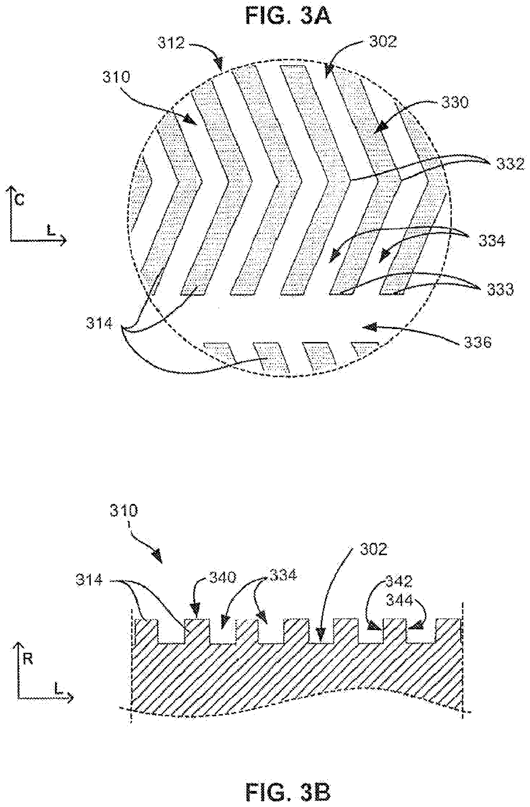

Figures 3A-3B disclose and describe examples of directional microtextures and nanotextures. Particular embodiments of the present invention include suture threads and other surgical filaments having thereon one or more of the directional microtexture and/or nanotextures described below.

FIGS. 3A and 3B show greatly enlarged views of a directional texture 310 which can be a directional microtexture and/or nanotexture depending upon the dimensions of the textural features 314. FIG. 3A shows a plan view of a patch 312 of a surface 302 having thereon the directional texture 310. FIG. 3A illustrate the distribution of the textural features 314 and a plan view of the shape of the textural features 314 along the longitudinal axis L and circumferential axis C. FIG. 3B shows a sectional view through normal to the surface 302. FIG. 3B illustrates aspect of the shape of the textural features 314 along the radial axis R. The orientation of the relevant axes is identified adjacent the Figures 3A and 3B.

Referring first to FIG. 3A which shows a plan view of a patch 312 of a surface 302 having thereon the directional microtexture and/or nanotexture 310 comprising an arrangement of textural features 314. In this embodiment, the textural features 314 are above surface 302 and textural features 314 are shaded in the drawing to distinguish them from textural surface 302. As shown in FIG. 3A directional texture 310 comprises a plurality of textural features 314 arranged in a regular pattern on surface 302. In plan view textural features 314 are in the shape of a chevron 330. Each chevron has an apex 332. The apices 332 of each chevron 330 are aligned along an axis parallel to the longitudinal axis L. Spaces between the textural features 314 are defined by textural features 314 as grooves 334 oriented at an angle to longitudinal axis L. Adjacent the ends of each chevron 330 is an area of surface 302 having no textural features 314. The area of surface 302 having no textural features 314 defines longitudinal grooves 336.

FIG. 3B shows a sectional view through surface 302 of directional texture 310 along the radial axis R and longitudinal axis L passing through textural features 314. As shown in FIG. 3B, Textural features 314 are approximately square in longitudinal section. Angled grooves 334 between textural features 314 are also square in section. The top 340 of textural features 314 is a flat surface approximately parallel to surface 302. The sides 342, 344 of textural features are vertical (normal) relative to surface 302.

In embodiments, the height of textural features 314 is less than about 10 µm. In preferred embodiments the height of textural features 314 is of the order of 1 µm. In alternative embodiments the height of textural features 314 is less than about 1 µm and greater than about 500 nanometers. The size of the textural features is significantly larger than the height in other dimensions. The width of each chevron 330 is for example, approximately an order of magnitude greater than the height of the textural features 314. In the embodiment shown in FIGS 3A, 3B directional texture 310 exhibits less resistance to passing through tissue in the forward direction along the longitudinal axis L (in the direction of deployment) compared to the reverse direction along the longitudinal axis L (against the direction of deployment) because of the shape, distribution and orientation of the textural features 314.

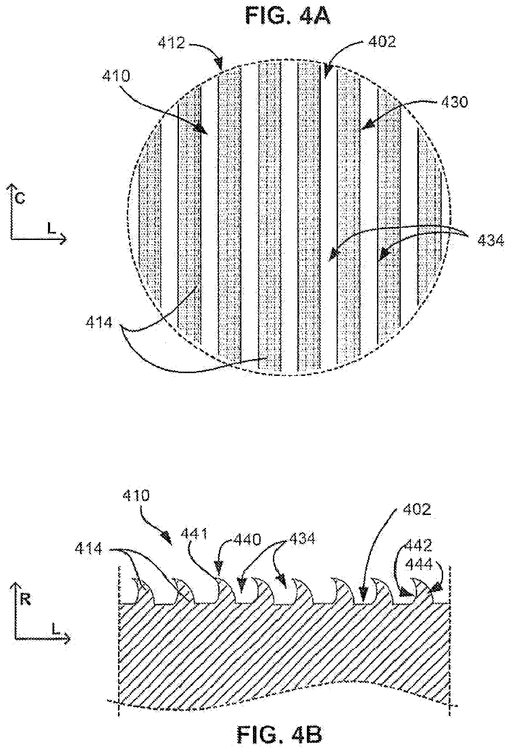

FIGS. 4A and 4B show greatly enlarged views of a directional texture 410 which can be a directional microtexture and/or nanotexture depending upon the dimensions of the textural features 414. FIG. 4A shows a plan view of a patch 412 of a surface 402 having thereon the directional texture 410. FIG. 4A illustrate the distribution of the textural features 414 and a plan view of the shape of the textural features 414 along the longitudinal axis L and circumferential axis C. FIG. 4B shows a sectional view through and normal to the surface 402. FIG. 4B illustrates aspects of the shape of the textural features 414 along the radial axis R. The orientation of the relevant axes is identified adjacent the Figures 4A and 4B.

Referring first to FIG. 4A which shows a plan view of a patch 412 of a surface 402 having thereon the directional microtexture and/or nanotexture 410 comprising an arrangement of textural features 414. In this embodiment, the textural features 414 are above surface 402 and textural features 414 are shaded in the drawing to distinguish them from textural surface 402. As shown in FIG. 4A directional texture 410 comprises a plurality of textural features 414 arranged in a regular pattern on surface 402. In plan view, textural features 414 are in the shape of circumferential ridges 430, perpendicular to the longitudinal axis L. Ridges 430 are, in some embodiments, continuous but in other embodiments ridges 430 are interrupted at intervals along their length by a gap (not shown). Ridges 430 are approximately parallel to one another. Spaces between the textural features 414 are defined by textural features 414 as grooves 434 oriented perpendicular to longitudinal axis L.

FIG. 4B shows a sectional view through surface 402 of directional texture 410 along the radial axis R and longitudinal axis L passing through textural features 414. As shown in FIG. 4B, Textural features 414 are wave-shaped in longitudinal section. Grooves 434 are defined by the shape of textural features 414 and generally increase in size moving away from surface 402. The top 440 of textural features 414 is a curved surface ending an apex 441 pointing to the left in FIG. 4B. Surface 442 of textural features 412 is concave, whereas surface 444 of textural features 412 is convex. Surfaces 442 and 444 meet at apex 441.

In embodiments, the height of textural features 414 is less than about 10 µm. In preferred embodiments the height of textural features 414 is of the order of 1µm. In alternative embodiments the height of textural features 414 is less than about 1 µm and greater than about 500 nanometers. The size of the textural features is significantly larger than the height in other dimensions. The width of each ridge 430 is for example, greater than an order of magnitude greater than the height of the textural features 414 and in some embodiments the width is then entire circumference of a suture thread. In the embodiment shown in FIGS 4A, 4B directional texture 410 exhibits less resistance to passing through tissue in the forward direction along the longitudinal axis L (in the direction of deployment) compared to the reverse direction along the longitudinal axis L (against the direction of deployment) because of the wave shape of the textural features 414.

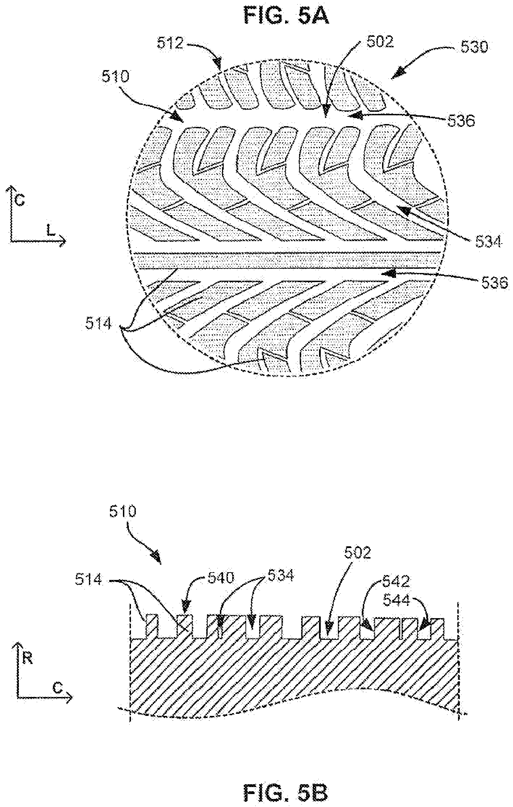

FIGS. 5A and 5B show greatly enlarged views of a directional texture 510 which can be a directional microtexture and/or nanotexture depending upon the dimensions of the textural features 514. FIG. 5A shows a plan view of a patch 512 of a surface 502 having thereon the directional texture 510. FIG. 5A illustrate the distribution of the textural features 514 and a plan view of the shape of the textural features 514 along the longitudinal axis L and circumferential axis C. FIG. 5B shows a sectional view through normal to the surface 502. FIG. 5B illustrates aspect of the shape of the textural features 514 along the radial axis R. The orientation of the relevant axes is identified adjacent the Figures 5A and 5B.

Referring first to FIG. 5A which shows a plan view of a patch 512 of a surface 502 having thereon the directional microtexture and/or nanotexture 510 comprising an arrangement of textural features 514. In this embodiment, the textural features 514 are above surface 502 and textural features 514 are shaded in the drawing to distinguish them from textural surface 502. As shown in FIG. 5A directional texture 510 comprises a plurality of textural features 514 arranged in a regular pattern on surface 502. In plan view textural features 514 have a plurality of irregular shapes. Additionally, one textural feature 514 is shaped as a ridge extending in the longitudinal direction. Spaces between the textural features 514 define angled grooves 534 oriented at an angle to longitudinal axis L and longitudinal grooves 536.

FIG. 5B shows a sectional view through surface 502 of directional texture 510 along the radial axis R and circumferential axis C passing through textural features 514. As shown in FIG. 5B, Textural features 514 are approximately rectangular/square in circumferential section. Angled grooves 534 between textural features 514 are also rectangular/square in section. The top 540 of textural features 514 is a flat surface approximately parallel to surface 502. The sides 542, 544 of textural features are vertical (normal) relative to surface 502.

In embodiments, the height of textural features 514 is less than about 10 µm. In preferred embodiments the height of textural features 514 is of the order of 1 µm. In alternative embodiments the height of textural features 514 is less than about a micron and greater than about 500 nanometers. The size of the textural features is significantly larger than the height in some dimensions. In the embodiment shown in FIGS 5A, 5B directional texture 510 exhibits less resistance to passing through tissue in the forward direction along the longitudinal axis L (in the direction of deployment) compared to the reverse direction along the longitudinal axis L (against the direction of deployment) because of the shape, distribution and orientation of the textural features 514.

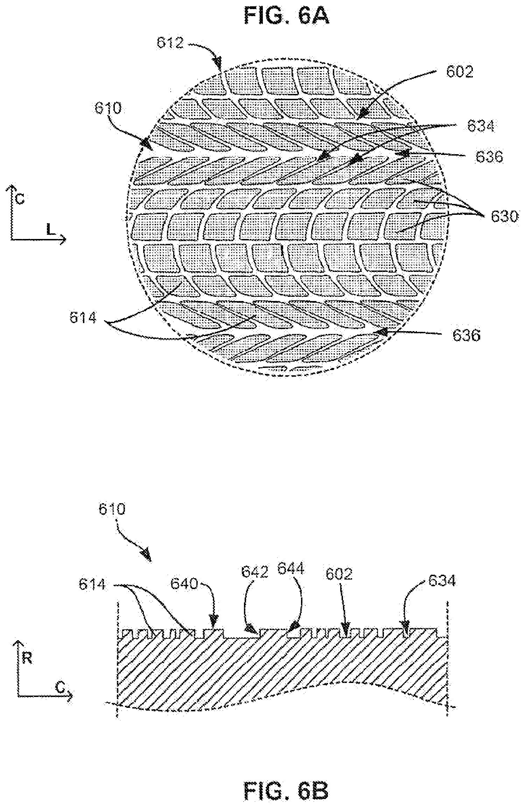

FIGS. 6A and 6B show greatly enlarged views of a directional texture 610 which can be a directional microtexture and/or nanotexture depending upon the dimensions of the textural features 614. FIG. 6A shows a plan view of a patch 612 of a surface 602 having thereon the directional texture 610. FIG. 6A illustrate the distribution of the textural features 614 and a plan view of the shape of the textural features 614 along the longitudinal axis L and circumferential axis C. FIG. 6B shows a sectional view through normal to the surface 602. FIG. 6B illustrates aspect of the shape of the textural features 614 along the radial axis R. The orientation of the relevant axes is identified adjacent the Figures 6A and 6B.

Referring first to FIG. 6A which shows a plan view of a patch 612 of a surface 602 having thereon the directional microtexture and/or nanotexture 610 comprising an arrangement of textural features 614. In this embodiment, the textural features 614 are above surface 602 and textural features 614 are shaded in the drawing to distinguish them from textural surface 602. As shown in FIG. 6A directional texture 610 comprises a plurality of irregular textural features 614 arranged in a repeating pattern on surface 602. In plan view textural features 614 have several different irregular shapes 630. The pattern of shapes 630 can be seen as creating a plurality of angled grooves 634 leading away from a plurality of vertical grooves 636.

FIG. 6B shows a sectional view through surface 602 of directional texture 610 along the radial axis R and longitudinal axis L passing through textural features 614. As shown in FIG. 6B, Textural features 614 are approximately square in longitudinal section. Grooves 636 between textural features 614 are also square in section. The top 640 of textural features 614 is a flat surface approximately parallel to surface 602. The sides 642, 644 of textural features are vertical (normal) relative to surface 602.

In embodiments, the height of textural features 614 is less than about 10 µm. In preferred embodiments the height of textural features 614 is of the order of 1 µm. In alternative embodiments the height of textural features 614 is less than about 1 µm and greater than about 500 nanometers. The size of the textural features is significantly larger than the height in other dimensions. The width of each chevron 630 is for example, approximately an order of magnitude greater than the height of the textural features 614. In the embodiment shown in FIGS 6A, 6B directional texture 610 exhibits less resistance to passing through tissue in the forward direction along the longitudinal axis L (in the direction of deployment) compared to the reverse direction along the longitudinal axis L (against the direction of deployment) because of the shape, distribution and orientation of the textural features 614.

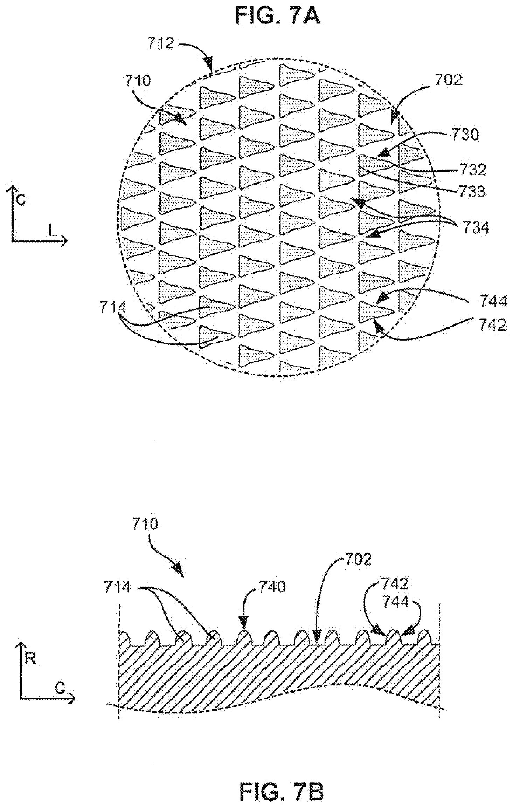

FIGS. 7A and 7B show greatly enlarged views of a directional texture 710 which can be a directional microtexture and/or nanotexture depending upon the dimensions of the textural features 714. FIG. 7A shows a plan view of a patch 712 of a surface 702 having thereon the directional texture 710. FIG. 7A illustrates the distribution of the textural features 714 and a plan view of the shape of the textural features 714 along the longitudinal axis L and circumferential axis C. FIG. 7B shows a sectional view through and normal to the surface 702. FIG. 7B illustrates aspects of the shape of the textural features 714 along the radial axis R. The orientation of the relevant axes is identified adjacent the Figures 7A and 7B.

Referring first to FIG. 7A which shows a plan view of a patch 712 of a surface 702 having thereon the directional microtexture and/or nanotexture 710 comprising an arrangement of textural features 714. In this embodiment, the textural features 714 are above surface 702 and textural features 714 are shaded in the drawing to distinguish them from textural surface 702. As shown in FIG. 7A directional texture 710 comprises a plurality of textural features 714 arranged in a regular pattern on surface 702. In plan view, textural features 714 are in the shape of triangles 730, aligned with the longitudinal axis L. Each triangle 730 has an apex 732 opposite a base 733. All of the apices 732 of triangles 730 point in the same direction along the longitudinal axis. The triangles 730 are aligned in rows parallel to the circumferential axis C. Spaces between the textural features 714 define angled grooves 734.

FIG. 7B shows a sectional view through surface 702 of directional texture 710 along the radial axis R and longitudinal axis L passing through textural features 714. As shown in FIG. 7B, Textural features 714 are rounded in circumferential section section. Grooves 734 are defined by the shape of textural features 714 and generally increase in size moving away from surface 702. The top 740 of textural features 714 is a curved surface ending. Surfaces 742 and 744 of textural feature 712 are both concave and meet at the top 740 of textural feature 714.

In embodiments, the height of textural features 714 is less than about 10 µm. In preferred embodiments the height of textural features 714 is of the order of 1 µm. In alternative embodiments the height of textural features 714 is less than about 1 µm and greater than about 500 nanometers. The size of the textural features is of similar size in other dimensions. In the embodiment shown in FIGS 7A, 7B directional texture 710 exhibits less resistance to passing through tissue in the forward direction along the longitudinal axis L (in the direction of deployment) compared to the reverse direction along the longitudinal axis L (against the direction of deployment) because of the triangle shape of the textural features 714.

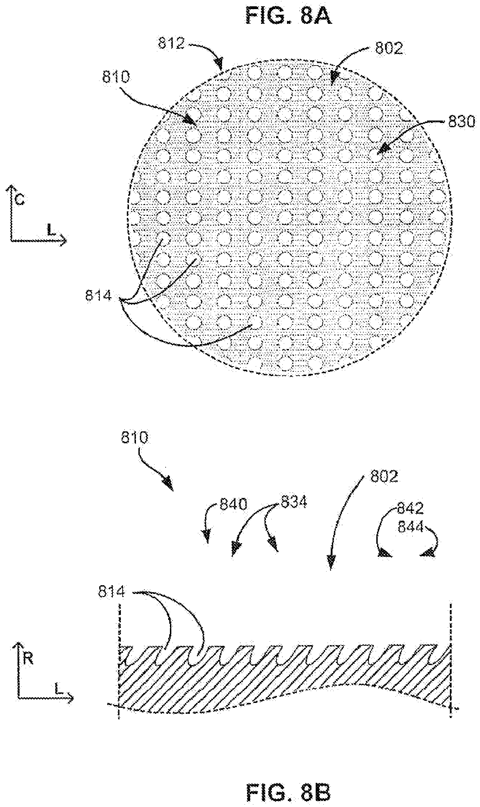

FIGS. 8A and 8B show greatly enlarged views of a directional texture 810 which can be a directional microtexture and/or nanotexture depending upon the dimensions of the textural features 814. FIG. 8A shows a plan view of a patch 812 of a surface 802 having thereon the directional texture 810. FIG. 8A illustrates the distribution of the textural features 814 and a plan view of the shape of the textural features 814 along the longitudinal axis L and circumferential axis C. FIG. 8B shows a sectional view through and normal to the surface 802. FIG. 8B illustrates aspects of the shape of the textural features 814 along the radial axis R. The orientation of the relevant axes is identified adjacent the Figures 8A and 8B.

Referring first to FIG. 8A which shows a plan view of a patch 812 of a surface 802 having thereon the directional microtexture and/or nanotexture 810 comprising an arrangement of textural features 814. In this embodiment, the textural features 814 are below surface 802 and surface 802 has been shaded to distinguish it from textural features 814. As shown in FIG. 8A directional texture 810 comprises a plurality of textural features 814 arranged in a regular pattern on surface 802. In plan view, textural features 814 are in the shape of circular pits 830, arranged in a grid. The circular pits 830 are aligned in rows parallel to the circumferential axis C and longitudinal axis L (although, in other embodiments the circular pits can be in offset or irregular arrangements along either or both axes). FIG. 8B shows a sectional view through surface 802 of directional texture 810 along the radial axis R and longitudinal axis L passing through textural features 814. As shown in FIG. 8B, Textural features 814 comprise tubes which are cut at an angle into surface 802.

In embodiments, the depth of textural features 814 is less than about 10 µm. In preferred embodiments the depth of textural features 814 is of the order of 1 µm. In alternative embodiments the depth of textural features 814 is less than about 1 µm and greater than about 500 nanometers. The diameter of textural features 814 is of similar size to the depth. In the embodiment shown in FIGS 8A, 8B directional texture 810 exhibits less resistance to passing through tissue in the forward direction along the longitudinal axis L (in the direction of deployment) compared to the reverse direction along the longitudinal axis L (against the direction of deployment) because of the angulation of circular pits 830.

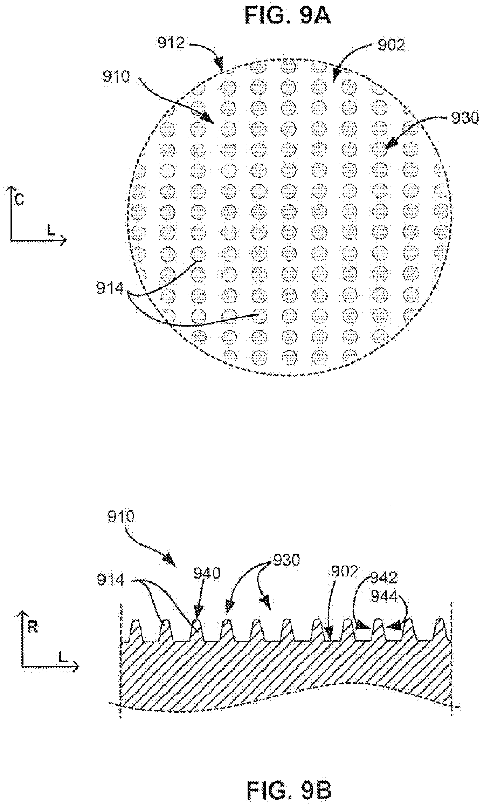

FIGS. 9A and 9B show greatly enlarged views of a texture 910 which can be a microtexture and/or nanotexture depending upon the dimensions of the textural features 914. FIG. 9A shows a plan view of a patch 912 of a surface 902 having thereon the texture 910. FIG. 9A illustrates the distribution of the textural features 914 and a plan view of the shape of the textural features 914 along the longitudinal axis L and circumferential axis C. FIG. 9B shows a sectional view through and normal to the surface 902. FIG. 9B illustrates aspects of the shape of the textural features 914 along the radial axis R. The orientation of the relevant axes is identified adjacent the Figures 9A and 9B.

Referring first to FIG. 9A which shows a plan view of a patch 912 of a surface 902 having thereon the directional texture 910 comprising an arrangement of textural features 914. In this embodiment, the textural features 914 are above surface 902 and have been shaded to distinguish them from surface 902. As shown in FIG. 9A texture 910 comprises a plurality of textural features 914 arranged in a regular pattern on surface 902. In plan view, textural features 914 are circular columns 930, arranged in a grid. The circular columns 930 are aligned in rows parallel to the circumferential axis C and longitudinal axis L (although, in other embodiments the circular pits can be in offset or irregular arrangements along either or both axes). FIG. 9B shows a sectional view through surface 902 of directional texture 910 along the radial axis R and longitudinal axis L passing through textural features 914. As shown in FIG. 9B, Textural features 914 comprise columns which are arranged substantially perpendicular to surface 902. In embodiments, the height of textural features 914 is less than about 10 µm. In preferred embodiments the height of textural features 914 is of the order of 1 µm. In alternative embodiments the height of textural features 914 is less than about 1 µm and greater than about 500 nanometers. The diameter of textural features 914 is of similar size to the height or smaller.

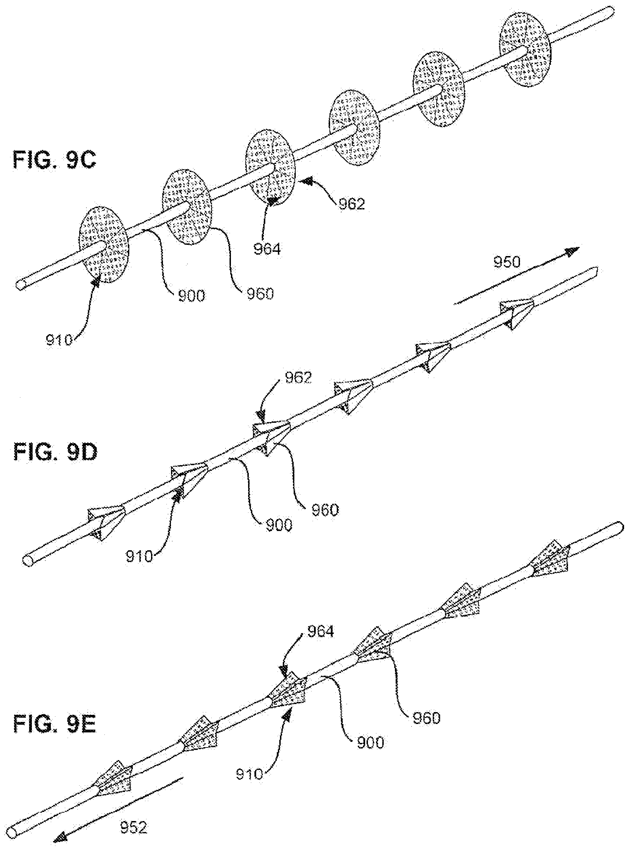

The microtexture/nanotexture shown in FIGS 9A, 9B has, in and of itself, the same resistance to passing through tissue in the forward direction along the longitudinal axis L (in the direction of deployment) compared to the reverse direction along the longitudinal axis L (against the direction of deployment) because the symmetrical arrangement and shape of circular columns 930. However, texture 910 can be used in a directional suture by providing texture 910 on surface which is not-exposed to tissue when the suture is moved in the direction of deployment but is exposed when the suture is moved in the reverse direction. In some embodiments, therefore, a surface microtexture and/or nanotexture having substantially equal resistance to movement of a suture in all directions can provided directional functionality when provided on a surface which is reconfigurable. FIGS. 9C-9E show an example of a reconfigurable surface bearing a microtexture and/or nanotexture.

FIG. 9C shows a suture thread 900 having thereon a plurality of flexible circular sheets 960. Sheets 960 are firmly attached at the center to suture thread 900. Sheets 960 are sufficiently thin and flexible that they collapse against suture thread 900 when suture thread 900 is deployed though tissue. Sheets 960 each have a proximal side 962 which is the side facing in the deployment direction. Sheets 960 each have a distal side 964 which is the side facing in the reverse direction. As shown in FIG. 9C, texture 910 is provided on the distal side 964 of sheets 960. No texture 910 is provided on the proximal side 962.

As, shown in FIG. 9D, when suture thread 900 is deployed through tissue in the deployment direction shown by arrow 950, sheets 960 collapse against suture thread 900 with the proximal sides 962 exposed. Texture 910 is protected from contact with tissue. Thus, suture thread 900 can move easily through tissue in the deployment direction 950. As shown in FIG. 9E, when suture thread 900 is deployed through tissue in the reverse direction shown by arrow 952, sheets 960 are reconfigured such that they collapse against suture thread 900 with the distal sides 962 exposed. Texture 910 is then brought into contact with tissue. The suture thread 900 thus resists movement through tissue in the reverse direction.

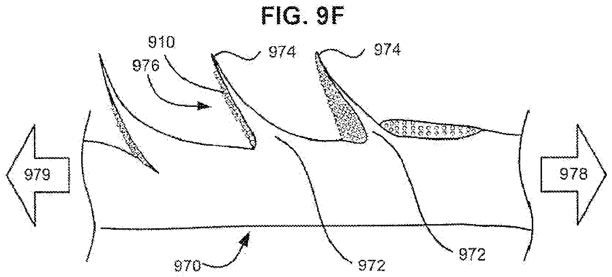

FIG. 9F illustrates the use of a microtexture and/or nano texture on a tissue engaging surface of a macro retainer, for example a barb. FIG. 9F shows a suture filament 970 on which is distributed a plurality of tissue retainers 972 in the form of barbs. Each tissue retainer has a tip 974 and a tissue engagement surface 976. The tissue retainers 972 are configured such that if the filament is moved through tissue in the deployment direction indicated by arrow 978, the tips 974 of the tissue retainers 972 move towards suture filament 970. Consequently, the suture filament has a low resistance to movement through tissue in the deployment direction. However, if the suture filament is moved in the reverse direction indicated by arrow 979, the tips 974 of tissue retainers 972 penetrate the tissue and move away from filament 970 bringing tissue engagement surfaces 976 in contact with the tissue. Consequently, the suture filament 970 has a high resistance to movement through tissue in the reverse direction 979.

As shown in FIG. 9F, in an embodiment, a surface microtexture and/or nanotexture 910 is provided selectively on the tissue engagement surfaces 976 of the tissue retainers 972. During deployment of the suture filament in direction 978, the surface microtexture and/or nanotexture 910 is protected from contact with tissue. However, when the tissue engagement surfaces 976 are brought into contact with tissue by movement of filament 970 in the reverse direction 799, the surface microtexture and/or nanotexture 910 is brought into contact with tissue and thereby augments the engagement of the tissue by tissue retainers 972.

In the embodiment of FIG. 9F, a non-directional surface microtexture and/or nanotexture has been selectively applied to a self-retaining suture to augment the function of the self-retaining suture. However, in alternative embodiments, one or more of the directional surface microtexture and/or nanotextures discussed herein is applied to the suture in a manner selected to facilitate movement through tissue in the deployment direction and/or resist movement through tissue in the reverse direction. In embodiments, the surface microtexture and/or nanotexture can be applied to the retainers 972 and/or the body of filament 970. In a bidirectional suture, the orientation and/or placement of the surface microtexture and/or nanotextures is applied separately to each arm of the suture in a manner selected to facilitate movement of that arm through tissue in the deployment direction and/or resist movement of that arm through tissue in the reverse direction.

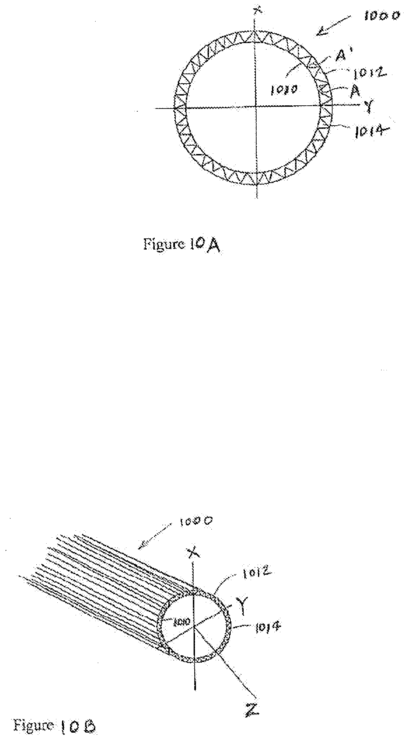

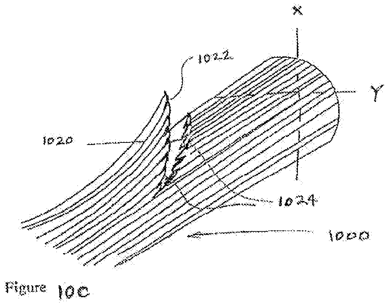

An embodiment of a self-retaining suture with retainers having enhanced tissue-penetrating capability is illustrated in FIGS. 10A, 10B, and 10C. Suture filament 1000 is shown, having surface features 1014 running along the filament 1000 substantially parallel to the longitudinal axis Z, which surface features may be microtextural features or nanotextural features, depending on their dimensions. As can be seen in the cross-sectional view shown in FIG. 10A and the cross-sectional perspective view of 10B, features 1014 may be considered either to be ridges on the surface 1010 of filament 1000 (created either by adding those ridges to an outer diameter 1012 or by extruding the filament material through a star-like dye) and having a height A, or grooves in the surface 1012 of filament 1000 (created by the selective removal of filament material to an inner diameter 1010) and having a depth A'. In either characterization, the height A and depth A' of the features in the X and Y axes are the same and are substantially fixed.

Referring now to FIG. 10C, it can be seen that a retainer 1020 cut into the textured filament 1000 (by, for example, a cutting blade, a cutting wheel, a laser) will have a tissue-penetrating edge 1022 with a variable thickness, correlating to factors such as the cut angle and distance between the outer edges of surface features 1014 and retainer cut surface 1024. This variable-thickness edge 1022 functions much like a serrated blade, thereby penetrating into tissue better than an edge having a uniform thickness._Of course, it is to be understood that retainers having enhanced tissue-penetrating edges can also be formed in micro- or nano-textured suture filaments having surface features that do not run parallel to the longitudinal axis of the suture; as long as the intersection of the surface features and the tissue-penetrating edge results in the edge having a variable thickness, the configuration and orientation of the surface features relative to the longitudinal axis is irrelevant.

### Manufacture Of Microtexture And Nanotexture

Textural features at the scale of 10 µm and smaller can be manufactured by a number of methods known in the art. Microtextured and nanotextured surfaces can be made by processes including, for example, extrusion, chemical vapor deposition, plasma etching, wet etching, EDM, nanomolding, stamping, printing, laser-cutting; laser ablation; imprint lithography. For example, methods for creating microtextures and nanotextures on generally planar surfaces are disclosed in the following references: U.S. Patent Publication No. 2009/0250588 entitled "Nanostructured Surfaces For Biomedical/Biomaterial Applications And Processes Therefore" to Robeson et al.; U.S. Patent Publication No. 20080131692 entitled "Methods And Materials For Fabricating Laminate Nanomolds And Nanoparticles Therefrom" to Rolland et al.; U.S. Patent Publication No. 2008/0169059 entitled "Biomimetic Modular Adhesive Complex: Materials, Methods And Applications Therefore" to Messersmith et al.; United States Patent Publication No. 2007/0282247 entitled "Medical Device Applications of Nanostructured Surfaces" to Desai et al.; United States Patent Publication 2008/0248216 entitled "Methods For Preparing Nanotextured Surfaces And Applications Thereof' to Yeung et al.; United States Patent Application 2009/0082856 entitled "Medical Devices Having Nanofiber-Textured Surfaces" to Flanagan; and Mahdave et al., "A biodegradable and biocompatible gecko-inspired tissue adhesive," PNAS 105 (7) 2307-2312 (2008); and Jeong et al. "A nontransferring dry adhesive with hierarchical polymer nanohairs," PNAS 106 (14) 5639-5644 (2009).

The above processes for creating nanotexture and microtextures on generally planar surfaces can be adapted in some respect for use on suture threads in a number of ways. In a simple embodiment, microtextured and or nanotextured films are created in a planar configuration and subsequently applied to the surface of a suture thread (see, e.g., FIGS. 9A-9E). Alternatively, nanomolding techniques can be adapted to treatment of continuous filaments utilizing rollers or similar rolling processes.

In preferred embodiments of the present invention, laser-cutting and/or laser ablating techniques are utilized to remove material from the surface of a suture thread to leave the desired microtexture and/or nanotexture. For example, excimer laser machining techniques are capable of creating features with a resolution of less than 10µm and femtosecond laser techniques (for example using a Ti:sapphire ultrafast laser) are capable of generating surface features at submicron resolution. Laser machining techniques are capable of cutting/ablating from drawn monofilaments. Monofilaments of drawn polymers are preferred materials because of their high tensile strength to diameter and their flexibility. Femtosecond laser techniques in particular are capable of forming features on monofilament sutures of USP 2-0 (0.3 mm) to 11-0 (0.01 mm) with resolutions smaller than 10µm, 5um, and 1um.

In alternative embodiments, material can be added to the surface of a suture thread to create microtexture and/or nanotexture on the surface of the thread. For example, a sheath of material is, in some embodiments, extruded over a suture thread. The sheath material is then manipulated/patterned using nanomolding, coining or photolithographic techniques to generate the desired texture and/or nanotexture.

### Coatings And Therapeutic Agents

In certain embodiments the surface of the suture is also provided with a therapeutic and/or adhesive agent/coating. For example, a surface microtexture and/or nanotexture in some embodiments benefits by treatment with a chemical that facilitates interaction with tissue. Thus, other substances and materials that it would be desirable to selectively expose to the tissue in similar fashion may be applied to the suture to facilitate their delivery to the desired targets. For example therapeutic compositions including, for example, compositions to promote healing and prevent undesirable effects such as scar formation, infection, pain, and so forth may be in some embodiments, advantageously incorporated with the material of the suture. In such embodiments the therapeutic composition is exposed to tissue at the inner retainer surface of the retainers. This arrangement is particularly suitable for therapeutic agents selected to promote and enhance the effectiveness of the retainer. Alternatively, the therapeutic agent may be selected to ameliorate any deleterious effects upon the tissue resulting from engagement of the tissue by the retainer and to promote healing. Compositions for incorporation in the suture, microtexture and/or nanotexture may include without limitation anti-proliferative agents, anti-angiogenic agents, anti-infective agents, fibrosis-inducing agents, anti-scarring agents, lubricious agents, echogenic agents, anti-inflammatory agents, cell cycle inhibitors, analgesics, and anti-microtubule agents.

The purpose of the suture may determine the sort of therapeutic agent that is applied to the suture; for example, self-retaining sutures having anti-proliferative agents may be used in closing tumor excision sites, while self-retaining sutures with fibrosing agents may be used in tissue repositioning procedures and those having anti-scarring agents may be used for wound closure on the skin. The compositions can be incorporated in the suture, microtexture and/or nanotexture in a variety of manners, including for example: (a) by directly affixing to the suture a formulation (e.g., by either spraying the suture with a polymer/drug film, or by dipping the suture into a polymer/drug solution) where the material is selectively absorbed by the suture, microtexture and/or nanotexture where exposed on the retainers, or (b) adding the composition to the raw material of the suture, microtexture and/or nanotexture layer prior to making the filament.

Therapeutic agents may also be coated on regions of the suture or the entire suture by spraying, dipping or absorption of the agent by a hydrogel applied to the suture surface. Coatings may also include a plurality of compositions either together or on different portions of the suture, where the multiple compositions can be selected either for different purposes (such as combinations of analgesics, anti-infective and anti-scarring agents) or for the synergistic effects of the combination. Compositions for incorporation in the suture, microtexture and/or nanotexture may include, without limitation anti-proliferative agents, anti-angiogenic agents, anti-infective agents, fibrosis-inducing agents, anti-scarring agents, lubricious agents, echogenic agents, anti-inflammatory agents, cell cycle inhibitors, analgesics, and anti-microtubule agents depending upon the purpose to which the suture will be put.

### Clinical Applications

In addition to the general wound closure and soft tissue repair applications, self-retaining sutures having surface microtexture and/or nanotextures can be used in a variety of other indications. Self-retaining sutures as described herein may be used in various dental procedures, i.e., oral and maxillofacial surgical procedures and thus may be referred to as "self-retaining dental sutures." Self-retaining sutures described herein may also be used in microsurgical procedures that are performed under a surgical microscope (and thus may be referred to as "self-retaining micro sutures"). Such surgical procedures include, but are not limited to, reattachment and repair of peripheral nerves, spinal microsurgery, microsurgery of the hand, various plastic microsurgical procedures (e.g., facial reconstruction), microsurgery of the male or female reproductive systems, and various types of reconstructive microsurgery. Microsurgical reconstruction is used for complex reconstructive surgery problems when other options such as primary closure, healing by secondary intention, skin grafting, local flap transfer, and distant flap transfer are not adequate. Self-retaining microsutures have a very small caliber, often as small as USP 8-0 (0,04 mm) to USP 11-0 (0,010 mm) and smaller. The microsutures may be degradable or non-degradable. Self-retaining sutures as described herein may be used in similarly small caliber ranges for ophthalmic surgical procedures and thus may be referred to as "ophthalmic self-retaining sutures". Such procedures include but are not limited to keratoplasty, cataract, and vitreous retinal microsurgical procedures. Self-retaining sutures can also be used in a variety of veterinary applications for a wide number of surgical and traumatic purposes in animal health.

Although the present invention has been shown and described in detail with regard to only a few exemplary embodiments of the invention, it should be understood by those skilled in the art that it is not intended to limit the invention to the specific embodiments disclosed. Various modifications, omissions, and additions may be made to the disclosed embodiments, particularly in light of the foregoing teachings. Accordingly, it is intended to cover all equivalents as may be included within the scope of the invention as defined by the following claims.

## Claims

1. A tissue retaining device comprising:
a flexible elongated suture thread having a surface (302, 502, 602), a longitudinal axis, a deployment direction along the longitudinal axis and a reverse direction, opposite to the deployment direction along the longitudinal axis;
the suture thread having distributed on at least a portion of the surface thereof a plurality of textural features (314), each of the textural features comprising a top surface (340) approximately parallel to the surface of the suture and having a height of between 500nm and 10µm; and
wherein the plurality of textural features includes one or more chevrons; and
wherein the plurality of textural features (314) cause the flexible elongated suture thread to have a greater resistance to movement through tissue in the reverse direction than in the deployment direction.

2. The tissue retaining device of claim 1, wherein:
the textural features (314) are asymmetrical with respect to a plane normal to the longitudinal axis of the suture thread thereby causing the flexible elongated suture thread to have a greater resistance to movement through tissue in the reverse direction than in the deployment direction.

3. The tissue retaining device of claim 1, wherein:
the suture thread has a first end, a second end, a periphery, and a plurality of retainers (972, 1020) projecting from the periphery of the body, wherein the plurality of retainers extend along a portion of the suture thread and are oriented in one direction; and
the textural features (314) are arranged in the portion of the suture thread.

4. The tissue retaining device of claim 1, wherein:
the suture thread has a first end, a second end, a periphery, and a plurality of retainers (972, 1020) projecting from the periphery of the body;
a first plurality of the retainers extend along a first portion of the suture thread and are oriented in one direction and a second plurality of the retainers extend along a second portion of the suture thread and are oriented in an opposite direction; and
the textural features (314) are arranged in a first orientation in the first portion of the suture thread and a second orientation, different than the first orientation, in the second portion of the suture thread.

5. The tissue retaining device of claim 1, wherein:
the suture thread has a first end, a second end, a periphery, and a plurality of retainers (972, 1020) projecting from the periphery of the body, each retainer (972, 1020) having a tissue-retaining surface oriented at an acute angle to the suture thread;
a first plurality of the retainers extend along a first portion of the suture thread and are oriented in one direction and a second plurality of the retainers extend along a second portion of the suture thread and are oriented in an opposite direction; and
the textural features (314) are arranged on the tissue-retaining surface of said retainers and are adapted to augment engagement of tissue by said tissue-retaining surface.

6. The tissue retaining device of claim 1, further comprising at least one retainer (972, 1020) on the portion of the suture thread surface, the at least one retainer being a cut at an acute angle into the suture thread body and including a variable-thickness tissue-penetrating edge oriented away from the first end of the suture thread.

7. The tissue-retaining device of claim 1, wherein the textural features (314) are oriented parallel to the longitudinal axis of the suture thread.

8. The tissue retaining device of claim 1, further comprising a plurality of retainers (972, 1020) on the elongated body, each retainer (972, 1020) having a tissue-penetrating edge oriented away from the first end and yielding toward the suture body during movement of the suture through tissue in a direction of deployment of the first end, and resisting movement of the suture, when in tissue, in a direction substantially opposite the direction of deployment of the first end.

9. The tissue retaining device of claim 1, further comprising:
a plurality of first retainers (972, 1020) disposed on the elongated body proximal to the first end, each first retainer (972, 1020) having a tissue-penetrating edge oriented away from the first end and yielding toward the suture body during movement of the suture through tissue in a direction of deployment of the first end, and resisting movement of the suture, when in tissue, in a direction substantially opposite the direction of deployment of the first end; and,
a plurality of second retainers disposed on the elongated body proximal to the second end, each second retainer having a tissue-penetrating edge oriented away from the second end and yielding toward the suture body during movement of the suture through tissue in a direction of deployment of the second end, and resisting movement of the suture, when in tissue, in a direction substantially opposite the direction of deployment of the second end;
the pluralities of first and second retainers being separated by a retainer-free portion of the suture thread.

10. The tissue retaining device of claim 1, further comprising at least one retainer (972, 1020) on the portion of the suture thread surface, the at least one retainer (972, 1020) being a cut at an acute angle into the suture thread body thereby providing a serrated tissue-penetrating edge oriented away from the first end of the suture thread.

## Patentansprüche

1. Gewebehaltevorrichtung, umfassend:
einen flexiblen länglichen Nahtfaden mit einer Oberfläche (302, 502, 602), einer Längsachse, einer Verwendungsrichtung entlang der Längsachse und einer umgekehrten Richtung, die der Verwendungsrichtung entlang der Längsachse entgegengesetzt ist;
wobei der Nahtfaden verteilt an mindestens einem Abschnitt der Oberfläche davon eine Vielzahl von Texturmerkmalen (314) aufweist, wobei jedes der Texturmerkmale eine Oberseite (340) umfasst, die ungefähr parallel zu der Oberfläche der Naht ist und eine Höhe zwischen 500nm und 10µm aufweist; und
wobei die Vielzahl von Texturmerkmalen ein oder mehrere Fischgrätmuster umfasst; und
wobei die Vielzahl von Texturmerkmalen (314) bewirkt, dass der flexible längliche Nahtfaden einen größeren Widerstand gegen die Bewegung durch Gewebe in der umgekehrten Richtung aufweist als in der Verwendungsrichtung.

2. Gewebehaltevorrichtung nach Anspruch 1, wobei:
die Texturmerkmale (314) mit Bezug auf eine Ebene normal zur Längsachse des Nahtfadens asymmetrisch sind, wodurch bewirkt wird, dass der flexible längliche Nahtfaden einen größeren Widerstand gegen die Bewegung durch Gewebe in der umgekehrten Richtung aufweist als in der Verwendungsrichtung.

3. Gewebehaltevorrichtung nach Anspruch 1, wobei:
der Nahtfaden ein erstes Ende, ein zweites Ende, eine Umfangslänge und eine Vielzahl von Haltern (972, 1020) aufweist, die von der Umfangslänge des Körpers vorspringen, wobei sich die Vielzahl von Haltern entlang eines Abschnitts des Nahtfadens erstreckt und in einer Richtung ausgerichtet ist; und
die Texturmerkmale (314) in dem Abschnitt des Nahtfadens angeordnet sind.

4. Gewebehaltevorrichtung nach Anspruch 1, wobei:
der Nahtfaden ein erstes Ende, ein zweites Ende, eine Umfangslänge und eine Vielzahl von Haltern (972, 1020) umfasst, die von der Umfangslänge des Körpers vorspringen;
sich eine erste Vielzahl der Halter entlang eines ersten Abschnitts des Nahtfadens erstreckt und in einer Richtung ausgerichtet ist und sich eine zweite Vielzahl der Halter entlang eines zweiten Abschnitts des Nahtfadens erstreckt und in einer entgegengesetzten Richtung ausgerichtet ist; und
die Texturmerkmale (314) in einer ersten Ausrichtung in dem ersten Abschnitt des Nahtfadens und einer zweiten Ausrichtung, die sich von der ersten Ausrichtung unterscheidet, in dem zweiten Abschnitt des Nahtfadens angeordnet sind.

5. Gewebehaltevorrichtung nach Anspruch 1, wobei:
der Nahtfaden ein erstes Ende, ein zweites Ende, eine Umfangslänge und eine Vielzahl von Haltern (972, 1020) aufweist, die von der Umfangslänge des Körpers vorspringen, wobei jeder Halter (972, 1020) eine Gewebehalteoberfläche aufweist, die in einem spitzen Winkel zu dem Nahtfaden ausgerichtet ist;
sich eine erste Vielzahl der Halter entlang eines ersten Abschnitts des Nahtfadens erstreckt und in einer Richtung ausgerichtet ist und sich eine zweite Vielzahl der Halter entlang eines zweiten Abschnitts des Nahtfadens erstreckt und in einer entgegengesetzten Richtung ausgerichtet ist; und
die Texturmerkmale (314) auf der Gewebehalteoberfläche der Halter angeordnet und dazu angepasst sind, den Eingriff ins Gewebe durch die Gewebehalteoberfläche zu verstärken.

6. Gewebehaltevorrichtung nach Anspruch 1, ferner umfassend mindestens einen Halter (972, 1020) an dem Abschnitt der Nahtfadenoberfläche, wobei der mindestens eine Halter ein Schnitt in einem spitzen Winkel in den Nahtfadenkörper ist und eine Gewebedurchdringungskante mit variabler Dicke umfasst, die von dem ersten Ende des Nahtfadens weg ausgerichtet ist.

7. Gewebehaltevorrichtung nach Anspruch 1, wobei die Texturmerkmale (314) parallel zur Längsachse des Nahtfadens ausgerichtet sind.

8. Gewebehaltevorrichtung nach Anspruch 1, ferner umfassend eine Vielzahl von Haltern (972, 1020) an dem länglichen Körper, wobei jeder Halter (972, 1020) eine Gewebedurchdringungskante aufweist, die von dem ersten Ende weg ausgerichtet ist und in Richtung des Nahtkörpers führt während der Bewegung der Naht durch das Gewebe in eine Richtung der Verwendung des ersten Endes, und der Bewegung der Naht in eine Richtung im Wesentlichen entgegen der Richtung der Verwendung des ersten Endes widersteht, wenn im Gewebe.

9. Gewebehaltevorrichtung nach Anspruch 1, ferner umfassend:
eine Vielzahl von ersten Haltern (972, 1020), die an dem länglichen Körper proximal zu dem ersten Ende angeordnet sind, wobei jeder erste Halter (972, 1020) eine Gewebedurchdringungskante aufweist, die von dem ersten Ende weg ausgerichtet ist und in Richtung des Nahtkörpers führt während der Bewegung der Naht durch das Gewebe in eine Richtung der Verwendung des ersten Endes, und der Bewegung der Naht in eine Richtung im Wesentlichen entgegengesetzt der Richtung der Verwendung des ersten Endes widersteht, wenn im Gewebe; und
eine Vielzahl zweiter Halter, die an dem länglichen Körper proximal zu dem zweiten Ende angeordnet sind, wobei jeder zweite Halter eine Gewebedurchdringungskante aufweist, die von dem zweiten Ende weg ausgerichtet ist und in Richtung des Nahtkörpers führt während der Bewegung der Naht durch das Gewebe in eine Richtung der Verwendung des zweiten Endes, und der Bewegung der Naht in eine Richtung im Wesentlichen entgegen der Richtung der Verwendung des zweiten Endes widersteht, wenn im Gewebe;
die Vielzahlen von ersten und zweiten Haltern durch einen Halter-freien Abschnitt des Nahtfadens getrennt sind.

10. Gewebehaltevorrichtung nach Anspruch 1, ferner umfassend mindestens einen Halter (972, 1020) an dem Abschnitt der Nahtfadenoberfläche, wobei der mindestens eine Halter (972, 1020) ein Schnitt in einem spitzen Winkel in den Nahtfadenkörper ist und eine gezackte Gewebedurchdringungskante liefert, die von dem ersten Ende des Nahtfadens weg ausgerichtet ist.

## Revendications

1. Dispositif de retenue de tissu, comprenant:
un fil de suture allongé souple présentant une surface (302, 502, 602), un axe longitudinal, une direction de déploiement le long de l'axe longitudinal et une direction inverse, opposée à la direction de déploiement le long de l'axe longitudinal;
le fil de suture présentant, distribuées sur au moins une partie de la surface de celui-ci une pluralité de caractéristiques texturales (314), chacune des caractéristiques texturales présentant une surface supérieure (340) approximativement parallèle à la surface de la suture et présentant une hauteur comprise entre 500 nm et 10 µm; et
dans lequel la pluralité de caractéristiques texturales comprennent un ou plusieurs chevron(s); et
dans lequel la pluralité de caractéristiques texturales (314) amènent le fil de suture allongé souple à présenter une plus grande résistance au déplacement à travers le tissu dans la direction inverse que dans la direction de déploiement.

2. Dispositif de retenue de tissu selon la revendication 1, dans lequel:
les caractéristiques texturales (314) sont asymétriques par rapport à un plan normal à l'axe longitudinal du fil de suture, amenant de ce fait le fil de suture allongé souple à présenter une plus grande résistance au déplacement à travers le tissu dans la direction inverse que dans la direction de déploiement.

3. Dispositif de retenue de tissu selon la revendication 1, dans lequel:
le fil de suture présente une première extrémité, une seconde extrémité, une périphérie et une pluralité de dispositifs de retenue (972, 1020) faisant saillie à partir de la périphérie du corps, dans lequel la pluralité de dispositifs de retenue s'étendent le long d'une partie du fil de suture et sont orientés dans une première direction; et
les caractéristiques texturales (314) sont agencées dans la partie du fil de suture.

4. Dispositif de retenue de tissu selon la revendication 1, dans lequel:
le fil de suture présente une première extrémité, une seconde extrémité, une périphérie et une pluralité de dispositifs de retenue (972, 1020) faisant saillie à partir de la périphérie du corps;
une première pluralité des dispositifs de retenue s'étendent le long d'une première partie du fil de suture et sont orientés dans une première direction, et une seconde pluralité des dispositifs de retenue s'étendent le long d'une seconde partie du fil de suture et sont orientés dans une direction opposée; et
les caractéristiques texturales (314) sont agencées dans une première orientation dans la première partie du fil de suture et dans une seconde orientation, différente de la première orientation, dans la seconde partie du fil de suture.

5. Dispositif de retenue de tissu selon la revendication 1, dans lequel
le fil de suture présente une première extrémité, une seconde extrémité, une périphérie et une pluralité de dispositifs de retenue (972, 1020) faisant saillie à partir de la périphérie du corps, chaque dispositif de retenue (972, 1020) présentant une surface de retenue de tissu orientée selon un angle aigu par rapport au fil de suture;
une première pluralité des dispositifs de retenue s'étendent le long d'une première partie du fil de suture et sont orientés dans une première direction, et une seconde pluralité des dispositifs de retenue s'étendent le long d'une seconde partie du fil de suture et sont orientés dans une direction opposée; et
les caractéristiques texturales (314) sont agencées sur la surface de retenue de tissu desdits dispositifs de retenue et sont adaptées pour renforcer l'engagement du tissu par ladite surface de retenue de tissu.

6. Dispositif de retenue de tissu selon la revendication 1, comprenant en outre au moins un premier dispositif de retenue (972, 1020) sur la partie de la surface du fil de suture, ledit au moins une premier dispositif de retenue étant une découpe suivant un angle aigu dans le corps de fil de suture et présentant un bord de pénétration de tissu d'épaisseur variable orientés à l'écart de la première extrémité du fil de suture.

7. Dispositif de retenue de tissu selon la revendication 1, dans lequel les caractéristiques texturales (314) sont orientées parallèlement à l'axe longitudinal du fil de suture.

8. Dispositif de retenue de tissu selon la revendication 1, comprenant en outre une pluralité de dispositifs de retenue (972, 1020) sur le corps allongé, chaque dispositif de retenue (972, 1020) présentant un bord de pénétration de tissu orienté à l'écart de la première extrémité et se fléchissant en direction du corps de suture pendant le déplacement de la suture à travers le tissu dans une direction de déploiement de la première extrémité, et résistant au déplacement de la suture lorsqu'elle se trouve dans le tissu, dans une direction sensiblement opposée à la direction de déploiement de la première extrémité.

9. Dispositif de retenue de tissu selon la revendication 1, comprenant en outre:
une pluralité de premiers dispositifs de retenue (972, 1020) disposés sur le corps allongé à proximité de la première extrémité, chacun des premiers dispositifs de retenue (972, 1020) présentant un bord de pénétration de tissu orienté à l'écart de la première extrémité et se fléchissant en direction du corps de suture pendant le déplacement de la suture à travers le tissu dans une direction de déploiement de la première extrémité, et résistant au déplacement de la suture, lorsqu'elle se trouve dans le tissu, dans une direction sensiblement opposée à la direction de déploiement de la première extrémité; et
une pluralité de seconds dispositifs de retenue disposés sur le corps allongé à proximité de la seconde extrémité, chacun des seconds dispositifs de retenue présentant un bord de pénétration de tissu orienté à l'écart de la seconde extrémité et se fléchissant en direction du corps de suture pendant le déplacement de la suture à travers le tissu dans une direction de déploiement de la seconde extrémité, et résistant au déplacement de la suture, lorsqu'elle se trouve dans le tissu, dans une direction sensiblement opposée à la direction de déploiement de la seconde extrémité;
les pluralités de premiers et de seconds dispositifs de retenue étant séparées par une partie exempte de dispositif de retenue du fil de suture.

10. Dispositif de retenue de tissu selon la revendication 1, comprenant en outre au moins un premier dispositif de retenue (972, 1020) sur la partie de la surface du fil de suture, ledit au moins un premier dispositif de retenue (972, 1020) étant une découpe suivant un angle aigu dans le corps de fil de suture, procurant de ce fait un bord dentelé de pénétration de tissu orienté à l'écart de la première extrémité du fil de suture.
